(19) Europäisches Patentamt
European Patent Office
Office européen des brevets

(11) EP 3 012 896 B1

(12) # EUROPEAN PATENT SPECIFICATION

(45) Date of publication and mention of the grant of the patent:
**28.03.2018 Bulletin 2018/13**

(21) Application number: **14813623.7**

(22) Date of filing: **17.06.2014**

(51) Int Cl.:
***H01M 10/0567*** *(2010.01)* ***C07C 69/96*** *(2006.01)*
***H01G 11/64*** *(2013.01)* ***H01M 6/16*** *(2006.01)*
***H01M 10/052*** *(2010.01)* ***H01M 10/0569*** *(2010.01)*

(86) International application number:
**PCT/JP2014/066071**

(87) International publication number:
**WO 2014/203912 (24.12.2014 Gazette 2014/52)**

(54) **NONAQUEOUS ELECTROLYTE SOLUTION, ELECTRICITY STORAGE DEVICE USING SAME, AND BIPHENYL GROUP-CONTAINING CARBONATE COMPOUND USED IN SAME**

LÖSUNG MIT WASSERFREIEM ELEKTROLYT, ELEKTRIZITÄTSSPEICHERUNGSVORRICHTUNG DAMIT UND DARIN VERWENDETE BIPHENYLGRUPPEHALTIGE CARBONATVERBINDUNG

SOLUTION D'ÉLECTROLYTE NON AQUEUX, DISPOSITIF D'ACCUMULATION D'ÉLECTRICITÉ UTILISANT CELLE-CI ET COMPOSÉ DE CARBONATE CONTENANT UN GROUPE BIPHÉNYLE UTILISÉ DANS CELLE-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2013 JP 2013130381**
**13.11.2013 JP 2013234498**

(43) Date of publication of application:
**27.04.2016 Bulletin 2016/17**

(73) Proprietor: **UBE Industries, Ltd.**
**Ube-shi, Yamaguchi 755-8633 (JP)**

(72) Inventor: **ABE, Koji**
**Ube-shi**
**Yamaguchi 755-8633 (JP)**

(74) Representative: **Gille Hrabal**
**Brucknerstrasse 20**
**40593 Düsseldorf (DE)**

(56) References cited:
**CH-A- 508 578**
**JP-A- 2000 058 115**
**JP-A- 2001 210 364**
**JP-A- 2001 332 297**
**JP-A- 2002 280 068**
**JP-A- 2004 111 169**
**JP-A- 2004 111 169**
**US-A1- 2002 001 756**
**US-A1- 2012 308 883**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to a nonaqueous electrolytic solution capable of improving electrochemical characteristics in a broad temperature range, especially capable of improving electrochemical characteristics at high temperatures and an energy storage device using the same.

### BACKGROUND ART

An energy storage device, especially a lithium secondary battery, has been widely used recently for a power source of a small-sized electronic device, such as a mobile telephone, a notebook personal computer, etc., and a power source for an electric vehicle or electric power storage. Since there is a possibility that such an electronic device or a vehicle is used in a broad temperature range, such as a high temperature in midsummer, a low temperature in an extremely cold region, etc., it is demanded to improve electrochemical characteristics with a good balance in a broad temperature range. In particular, in the case where a laminate-type battery or a prismatic battery which is used in a thin electronic device, such as a tablet terminal, an ultrabook, etc., is used at high temperatures, an outer packaging member is thin, and therefore, there is involved such a problem that the battery is easily deformed by even a bit of expansion or the like, so that the deformation very likely influences the electronic device.

In addition, in order to prevent the global warming, it is an urgent need to reduce the $CO_2$ emission. Among eco-friendly vehicles mounted with an energy storage apparatus composed of an energy storage device, such as lithium secondary batteries, capacitors, etc., early dissemination of a hybrid electric vehicle (HEV), a plug-in hybrid electric vehicle (PHEV), or a battery electric vehicle (BEV) is demanded.

Since a vehicle is long in moving distance, there is a possibility that the vehicle is used in regions in a broad temperature range from a very hot region of the torrid zone to an extremely cold region. In consequence, in particular, these onboard energy storage devices are required such that even when used in a broad temperature range from high temperatures to low temperatures, the electrochemical characteristics are not worsened.

In the present specification, the term, lithium secondary battery, is used as a concept also including a so-called lithium ion secondary battery.

A lithium secondary battery is mainly constituted of a positive electrode and a negative electrode, each containing a material capable of absorbing and releasing mainly lithium, and a nonaqueous electrolytic solution containing a lithium salt and a nonaqueous solvent, and a carbonate, such as ethylene carbonate (EC), propylene carbonate (PC), etc., is used as the nonaqueous solvent.

In addition, metal lithium, a metal compound capable of absorbing and releasing lithium (e.g., a metal elemental substance, a metal oxide, an alloy with lithium, etc.), and a carbon material are known as the negative electrode. In particular, a lithium secondary battery using a carbon material capable of absorbing and releasing lithium, such as coke, artificial graphite, natural graphite, etc., is widely put into practical use.

For example, in a lithium secondary battery using, as the negative electrode material, a highly crystallized carbon material, such as natural graphite, artificial graphite, etc., it is known that a decomposition product or gas generated by reductive decomposition of a solvent in a nonaqueous electrolytic solution on the negative electrode surface on charging hinders a desired electrochemical reaction of the battery, so that worsening of cycle property is possibly caused. When decomposition products of the nonaqueous solvent are accumulated, absorption and release of lithium on the negative electrode may not be performed smoothly, and the electrochemical characteristics when used in a broad temperature range are liable to be worsened. Since the aforementioned negative electrode material absorbs and releases lithium and an electron at an extremely electronegative potential equal to the lithium metal, there were involved such problems that there is a possibility that a large number of solvents are liable to be subjected to reductive decomposition especially at high temperatures, and the movement of a lithium ion is hindered due to deposition of a decomposition product, gas generation, or expansion of the electrode, thereby not only worsening cycle property but also causing deformation of the battery.

Furthermore, it is known that a lithium secondary battery using a lithium metal or an alloy thereof, or a metal elemental substance, such as tin, silicon, etc., or an oxide thereof as the negative electrode material may have a high initial battery capacity, but the battery performance thereof, such as battery capacity and cycle property, may be largely worsened because the micronized powdering may be promoted during cycles, thereby bringing about accelerated reductive decomposition of the nonaqueous solvent, as compared with the negative electrode formed of a carbon material. When such a negative material is micronized, or decomposition products of the nonaqueous solvent are accumulated, absorption and release of lithium on the negative electrode may not be performed smoothly, and the electrochemical characteristics when used in a broad temperature range are liable to be worsened. It is known that a problem, such as significant worsening of battery performances, e.g., battery capacity and cycle property, deformation of the battery due

to expansion of the electrode, etc., is caused especially at high temperatures.

Meanwhile, it is known that in a lithium secondary battery using, as a positive electrode material, for example, $LiCoO_2$, $LiMn_2O_4$, $LiNiO_2$, $LiFePO_4$, etc., on an interface between the positive electrode material and the nonaqueous electrolytic solution in such a state that the nonaqueous solvent in the nonaqueous electrolytic solution is charged, a decomposition product or gas generated by a partial oxidative decomposition which is caused locally hinders a desired electrochemical reaction of the battery, and therefore, the electrochemical characteristics when used in a broad temperature range are liable to be worsened, too. There were involved such problems that there is a possibility that a large number of solvents are liable to be subjected to oxidative decomposition especially at high temperatures, and the movement of a lithium ion is hindered irrespective of the kind of the positive electrode material, thereby worsening battery characteristics, such as cycle property, etc.

In the light of the above, the battery performance was worsened due to the matter that the movement of a lithium ion is hindered or the battery is expanded by a decomposition product or gas when the nonaqueous electrolytic solution is decomposed on the positive electrode or negative electrode. Irrespective of such a situation, the multifunctionality of electronic devices on which lithium secondary batteries are mounted is more and more advanced, and power consumption tends to increase. For that reason, the capacity of lithium secondary battery is thus being much increased, and the space volume for the nonaqueous electrolytic solution in the battery is decreased by increasing the density of the electrode, or reducing the useless space volume in the battery, or the like. In consequence, it is a situation that the electrochemical characteristics when used in a broad temperature range, especially at high temperatures, are liable to be worsened due to even a bit of decomposition of the nonaqueous electrolytic solution.

PTL 1 proposes a nonaqueous electrolytic solution containing an aromatic carbonic acid ester, such as bis(4-phenylphenyl) carbonate, etc., and a nonaqueous electrolytic solution secondary battery using the same, and suggests that the safety on overcharging is improved.

## CITATION LIST

### PATENT LITERATURE

PTL 1: JP-A 2004-111169

## SUMMARY OF INVENTION

### TECHNICAL PROBLEM

A problem of the present invention is to provide a nonaqueous electrolytic solution capable of improving electrochemical characteristics in a broad temperature range, especially at high temperatures and also reducing a rate of increase in electrode thickness after high-temperature cycles and an energy storage device using the same.

### SOLUTION TO PROBLEM

The present inventors made extensive and intensive investigations regarding the performances of the nonaqueous electrolytic solution of the aforementioned conventional technique, and noted that the safety on overcharging may be improved to some extent in the nonaqueous electrolytic solution secondary battery of PTL 1, but PTL 1 does not disclose anything about a problem of reducing the rate of increase in electrode thickness following the charge and discharge.

Then, in order to solve the foregoing problem, the present inventors made extensive and intensive investigations. As a result, it has been found that by incorporating a specified biphenyl group-containing carbonate compound in a nonaqueous electrolytic solution, the rate of increase in electrode thickness after high-temperature cycles can be reduced, thereby leading to accomplishment of the present invention.

Specifically, the present invention is provided in the claims.

### ADVANTAGEOUS EFFECTS OF INVENTION

According to the present invention, it is possible to provide a nonaqueous electrolytic solution capable of improving electrochemical characteristics in a broad temperature range, especially a nonaqueous electrolytic solution capable of reducing a rate of increase in electrode thickness after high-temperature cycles and an energy storage device using the same, such as a lithium battery.

DESCRIPTION OF EMBODIMENTS

[Nonaqueous Electrolytic Solution]

The nonaqueous electrolytic solution of the present invention is a nonaqueous electrolytic solution having an electrolyte salt dissolved in a nonaqueous solvent, the nonaqueous electrolytic solution containing a biphenyl group-containing carbonate compound represented by the following general formula (I):

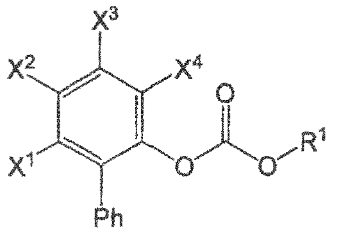

(I)

wherein $R^1$ represents an alkyl group having 1 to 12 carbon atoms, which may be substituted with a halogen atom, an alkenyl group having 2 to 6 carbon atoms, an alkynyl group having 3 to 6 carbon atoms, or an aryl group having 6 to 20 carbon atoms, which may be substituted with a halogen atom; and each of $X^1$ to $X^4$ independently represents a hydrogen atom, a phenyl group, or a benzyl group, wherein the nonaqueous electrolytic solution further comprises a fluorine atom-containing cyclic carbonate or an unsaturated bond-containing cyclic carbonate.

Although the reason why the nonaqueous electrolytic solution of the present invention is capable of improving electrochemical characteristics in a broad temperature range, especially at high temperatures and also reducing a rate of increase in electrode thickness after high-temperature cycles is not necessarily clear, the following may be considered.

It may be considered that the nonaqueous electrolytic solution of the present invention forms a mixture surface film containing at least three characteristic groups. That is, (i) in the case where in the compound represented by the general formula (I), at least one of $X^1$ to $X^4$ is a phenyl group or a benzyl group, the compound represented by the general formula (I) is subjected to oxidative reduction, a polymerization reaction rapidly proceeds, and a mixture surface film containing at least three characteristic groups of "biphenyl group", "phenyl group or benzyl group", and "-O-C(=O)-O- group" is liable to be formed. In addition, (ii) in the case where in the compound represented by the general formula (I), all of $X^1$ to $X^4$ are a hydrogen atom, by jointly using a fluorine atom-containing or an unsaturated bond-containing cyclic carbonate, a mixture surface film (SEI surface film) containing at least three characteristic groups of "biphenyl group", "-O-C(=O)-O- group", and "fluorine atom or unsaturated bond" is liable to be formed on the electrode interface. It may be considered that in the case where the substitution position of the benzene ring in the general formula (I) is an ortho position relative to the carbonate skeleton, such a mixture surface film becomes firm and has appropriate flexibility to such an extent that the permeation of a lithium ion is not disturbed, and it may be considered that further decomposition of the solvent is suppressed, whereby an increase in electrode thickness after high-temperature cycles may be much more suppressed. It may be considered that the mixture surface film containing these characteristic groups is formed of one or more compounds.

The biphenyl group-containing carbonate compound which is contained in the nonaqueous electrolytic solution of the present invention is represented by the following general formula (I).

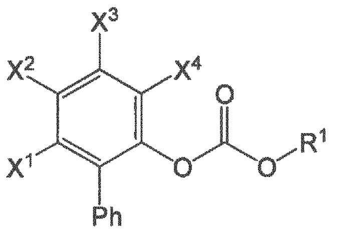

(I)

wherein $R^1$ represents an alkyl group having 1 to 12 carbon atoms, which may be substituted with a halogen atom, an alkenyl group having 2 to 6 carbon atoms, an alkynyl group having 3 to 6 carbon atoms, or an aryl group having 6 to 20 carbon atoms, which may be substituted with a halogen atom; and each of $X^1$ to $X^4$ independently represents a hydrogen atom, a phenyl group, or a benzyl group.

In the foregoing general formula (I), $R^1$ is preferably an alkyl group having 1 to 3 carbon atoms, an alkenyl group having 2 to 3 carbon atoms, an alkynyl group having 3 to 4 carbon atoms, or an aryl group having 6 to 18 carbon

atoms, and more preferably an alkyl group having 1 or 2 carbon atoms, an alkenyl group having 3 carbon atoms, an alkynyl group having 3 carbon atoms, or an aryl group having 6 to 12 carbon atoms.

As specific examples of $R^1$, there are suitably exemplified a straight-chain alkyl group, such as a methyl group, an ethyl group, an n-propyl group, an n-butyl group, an n-pentyl group, an n-hexyl group, an n-heptyl group, an n-octyl group, an n-decyl group, an n-dodecyl group, etc.; a branched alkyl group, such as an isopropyl group, a sec-butyl group, a tert-butyl group, a tert-amyl group, a 2-ethylhexyl group, etc.; a cycloalkyl group, such as a cyclopentyl group, a cyclohexyl group, etc.; a straight-chain alkenyl group, such as a vinyl group, a 2-propenyl group, a 2-butenyl group, a 3-butenyl group, etc.; a branched alkenyl group, such as a 2-methyl-2-propenyl group, etc.; a straight-chain alkynyl group, such as a 2-propynyl group, a 2-butynyl group, a 3-butynyl group, a 4-pentynyl group, a 5-hexynyl group, etc.; a branched alkynyl group, such as a 1-methyl-2-propynyl group, a 1-methyl-2-butynyl group, a 1,1-dimethyl-2-propynyl group, etc.; a fluoroalkyl group, such as a fluoromethyl group, a difluoromethyl group, a trifluoromethyl group, a 2-fluoroethyl group, a 2,2-difluoroethyl group, a 2,2,2-trifluoroethyl group, etc.; and an aryl group, such as a phenyl group, a 2-methylphenyl group, a 2,4-dimethylphenyl group, a 2,5-dimethylphenyl group, a 3-methylphenyl group, a 4-methylphenyl group, a 4-tert-butylphenyl group, a 2-fluorophenyl group, a 4-fluorophenyl group, a 4-trifluoromethylphenyl group, a 2,4-difluorophenyl group, a perfluorophenyl group, a 2-phenylphenyl group, a 3-phenylphenyl group, a 4-phenylphenyl group, a 2,6-diphenylphenyl group, etc.

Of those, a methyl group, an ethyl group, an n-propyl group, an isopropyl group, a vinyl group, a 2-propenyl group, a 2-propynyl group, a 2-butynyl group, a 3-butynyl group, a phenyl group, a 2-phenylphenyl group, a 3-phenylphenyl group, a 4-phenylphenyl group, a 2,6-diphenylphenyl group, a 2,4-dimethylphenyl group, a 2-fluorophenyl group, and a perfluorophenyl group are preferred; and a methyl group, an ethyl group, a 2-propenyl group, a 2-propynyl group, a phenyl group, a 2-phenylphenyl group, a 3-phenylphenyl group, a 4-phenylphenyl group, and a 2,6-diphenylphenyl group are more preferred.

In the foregoing general formula (I), each of $X^1$ to $X^4$ independently represents a hydrogen atom, a phenyl group, or a benzyl group; preferably, $X^2$ is a hydrogen atom; and more preferably, all of $X^1$ to $X^3$ are a hydrogen atom.

In the foregoing general formula (I), in the case where at least one of $X^1$ to $X^4$ is a phenyl group or a benzyl group, specifically, there are suitably exemplified the following compounds.

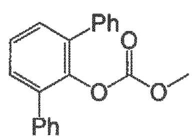
A1

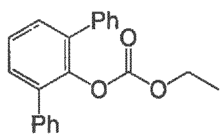
A2

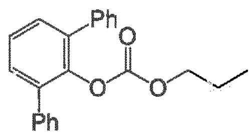
A3

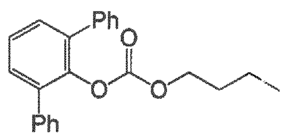
A4

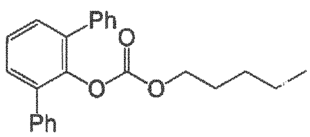
A5

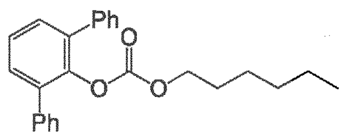
A6

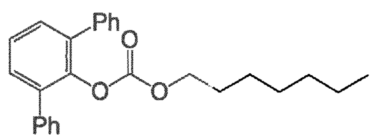
A7

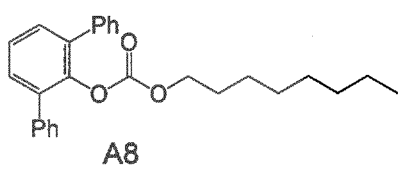
A8

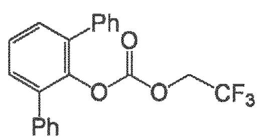
A9

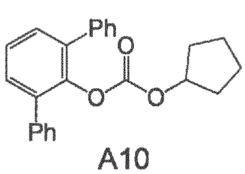
A10

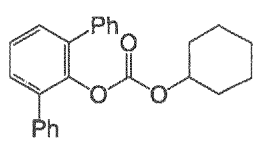
A11

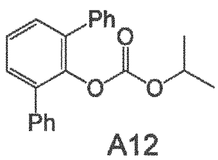
A12
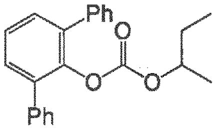
A13
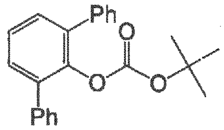
A14
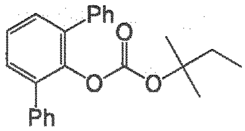
A15

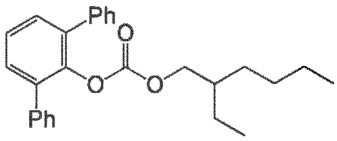
A16
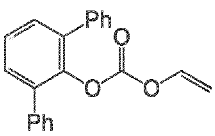
A17
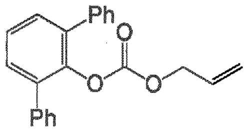
A18

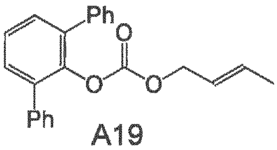
A19
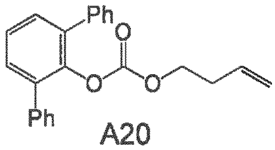
A20
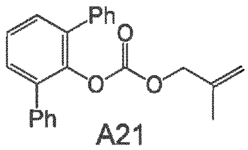
A21

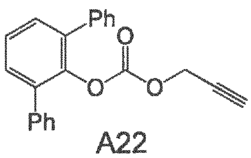
A22
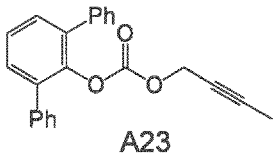
A23

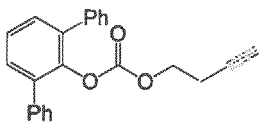
A24
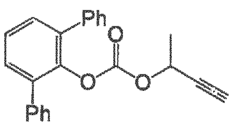
A25
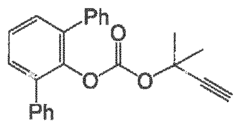
A26

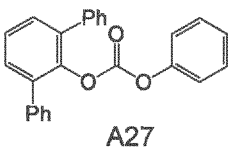
A27
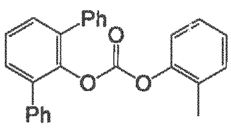
A28
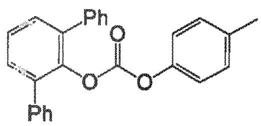
A29
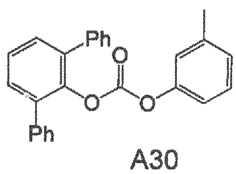
A30

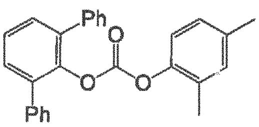
A31
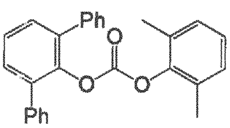
A32
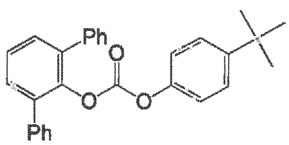
A33

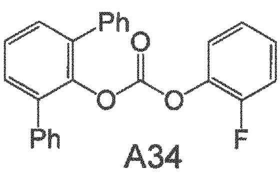
A34
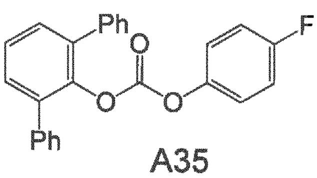
A35
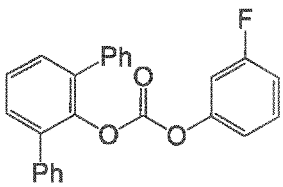
A36

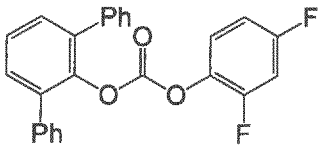
A37

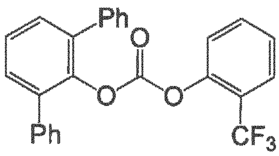
A38

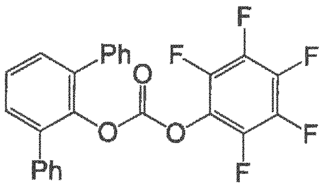
A39

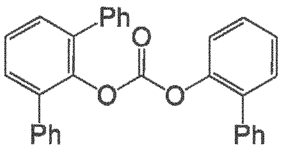
A40

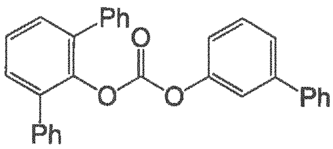
A41

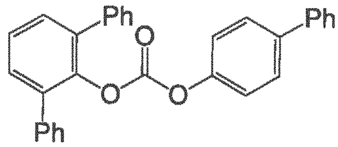
A42

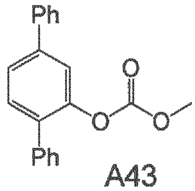
A43

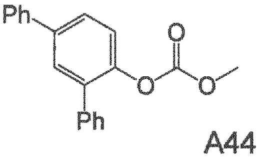
A44

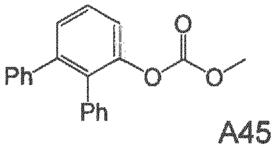
A45

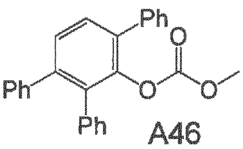
A46

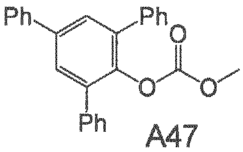
A47

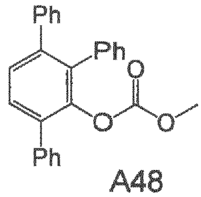
A48

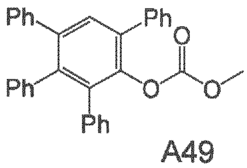
A49

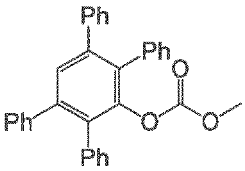
A50

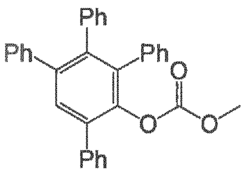
A51

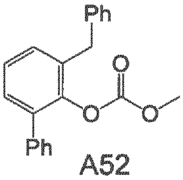
A52

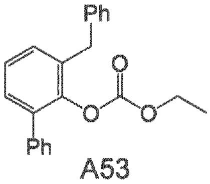
A53

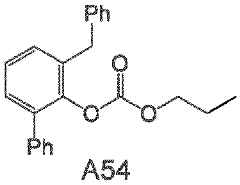
A54

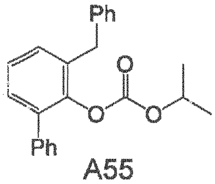
A55

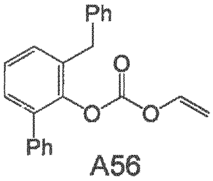
A56

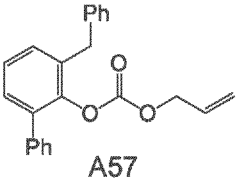
A57

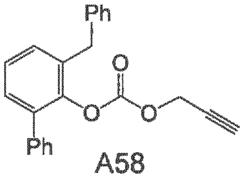
A58

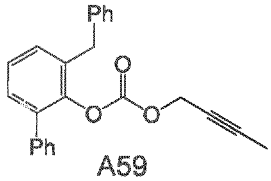
A59

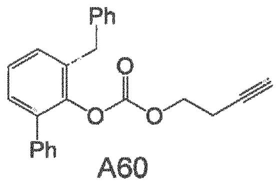
A60
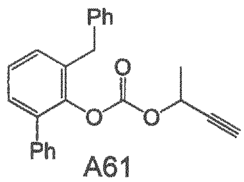
A61
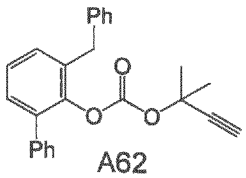
A62

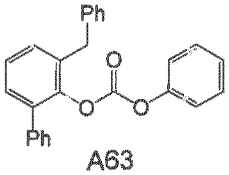
A63
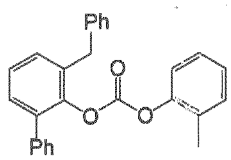
A64
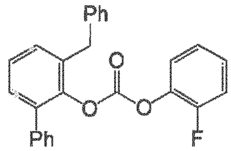
A65
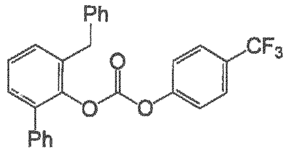
A66

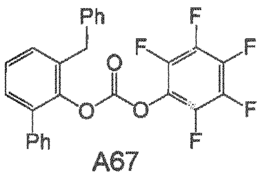
A67
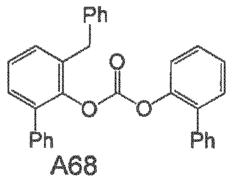
A68
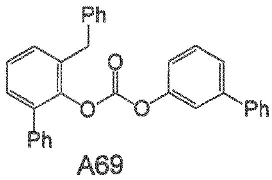
A69
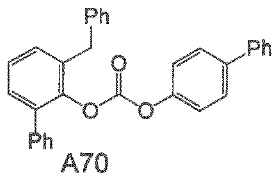
A70

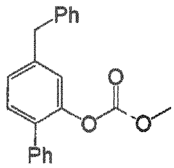
A71
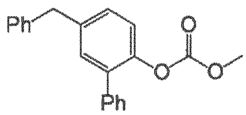
A72
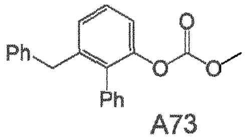
A73

Of the foregoing compounds, compounds having a structure in which any one of $X^1$ to $X^4$ in the general formula (I) is a phenyl group or a benzyl group, such as Compounds A1 to A45, and A52 to A73, etc., are preferred; compounds having a structure in which $X^4$ is a phenyl group or a benzyl group are more preferred; and one or more selected from 2,6-diphenylphenyl methyl carbonate (Compound A1), 2,6-diphenylphenyl ethyl carbonate (Compound A2), 2,6-diphenylphenyl allyl carbonate (Compound A18), 2,6-diphenylphenyl 2-propynyl carbonate (Compound A22), 2,6-diphenylphenyl phenyl carbonate (Compound A27), 2,6-diphenylphenyl 2-phenylphenyl carbonate (Compound A40), 2,6-diphenylphenyl 3-phenylphenyl carbonate (Compound A41), 2,6-diphenylphenyl 4-phenylphenyl carbonate (Compound A42), 2,4-diphenylphenyl carbonate (Compound A44), 2-benzyl-6-phenylphenyl methyl carbonate (Compound A52), 2-benzyl-6-phenylphenyl ethyl carbonate (Compound A53), 2-benzyl-6-phenylphenyl allyl carbonate (Compound A57), 2-benzyl-6-phenylphenyl 2-propynyl carbonate (Compound A58), 2-benzyl-6-phenylphenyl phenyl carbonate (Compound A63), 2-benzyl-6-phenylphenyl 2-phenylphenyl carbonate (Compound A68), 2-benzyl-6-phenylphenyl 3-phenylphenyl carbonate (Compound A69), and 2-benzyl-6-phenylphenyl 4-phenylphenyl carbonate (Compound A70) are still more preferred. This is because when the substitution position of the phenyl group or benzyl group is $X^4$, a surface film which is firm and has appropriate flexibility to such an extent that the permeation of a lithium ion is not disturbed is liable to be formed, and an increase in electrode thickness can be suppressed.

In the foregoing general formula (I), in the case where all of $X^1$ to $X^4$ are a hydrogen atom, specifically, there are suitably exemplified the following compounds.

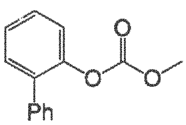
B1
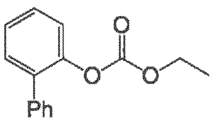
B2
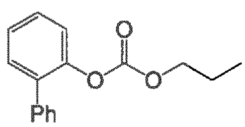
B3
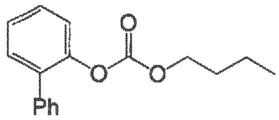
B4

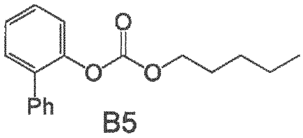
B5

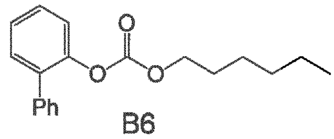
B6

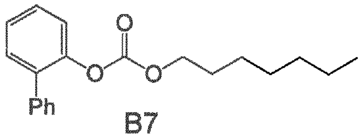
B7

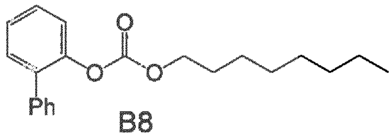
B8

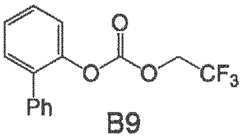
B9

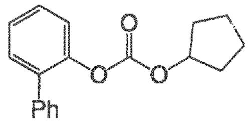
B10

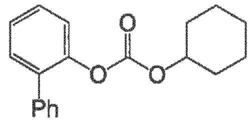
B11

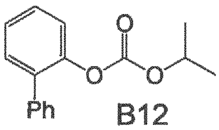
B12

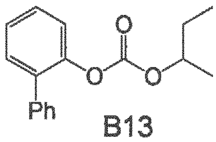
B13

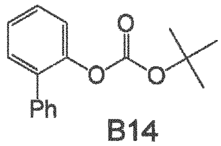
B14

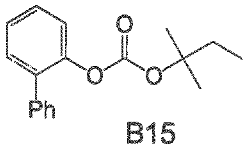
B15

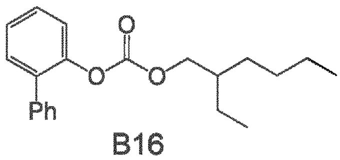
B16

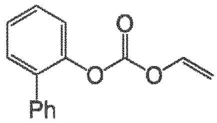
B17

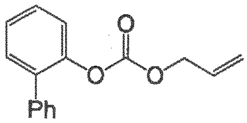
B18

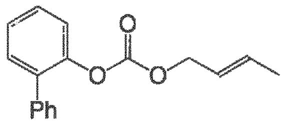
B19

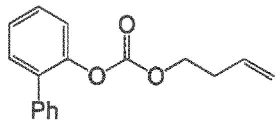
B20

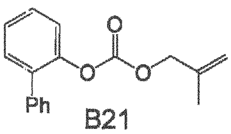
B21

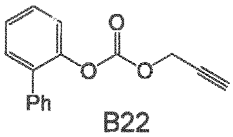
B22

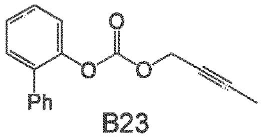
B23

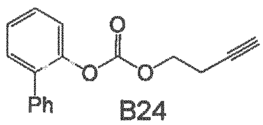
B24

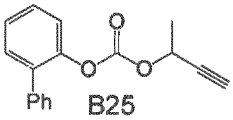
B25

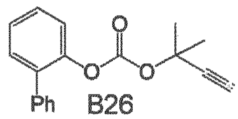
B26

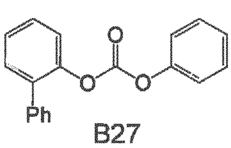
B27

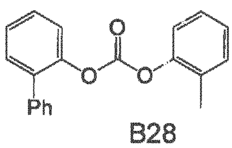
B28

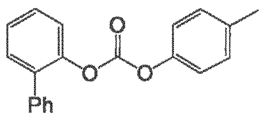
B29

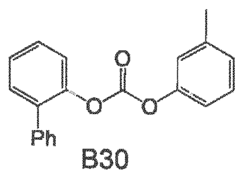
B30

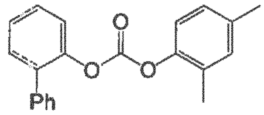
B31

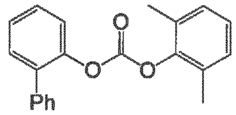
B32

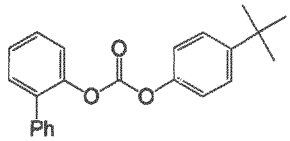
B33

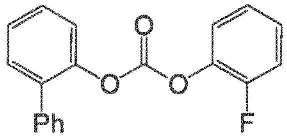
B34

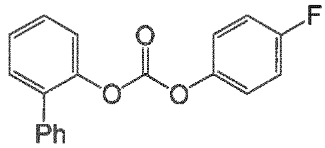
B35

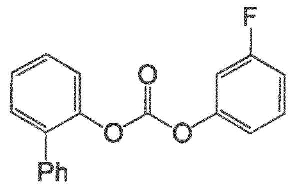
B36

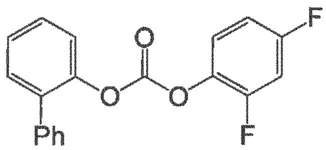
B37

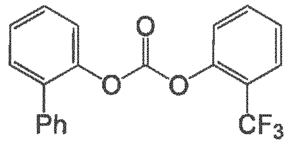
B38

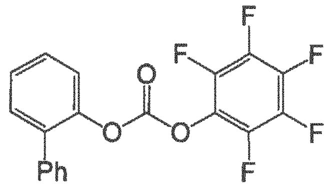
B39

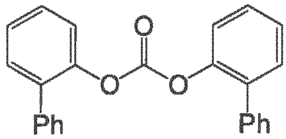
B40

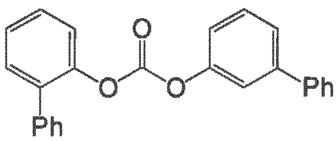
B41

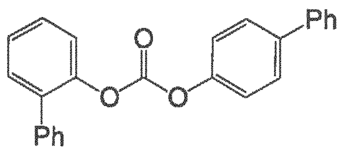
B42

Of the foregoing compounds, compounds having structures of Compounds B1 to B4, B17 to B18, B22, B27, and B39 to B42 are preferred; and one or more selected from methyl 2-phenylphenyl carbonate (Compound B1), ethyl 2-phenylphenyl carbonate (Compound B2), allyl 2-phenylphenyl carbonate (Compound B18), 2-phenylphenyl 2-propynyl carbonate (Compound B22), phenyl 2-phenylphenyl carbonate (Compound B27), bis(2-phenylphenyl) carbonate (Compound B40), 2-phenylphenyl 3-phenylphenyl carbonate (Compound B41), and 2-phenylphenyl 4-phenylphenyl carbonate (Compound B42) are more preferred. This is because by using such a compound, a surface film which is firm and has appropriate flexibility to such an extent that the permeation of a lithium ion is not disturbed is liable to be formed, and an increase in electrode thickness can be suppressed.

In the nonaqueous electrolytic solution of the present invention, a content of the biphenyl group-containing carbonate compound represented by the foregoing general formula (I), which is contained in the nonaqueous electrolytic solution, is preferably 0.001 to 5% by mass in the nonaqueous electrolytic solution. So long as the content is 5% by mass or less, there is less concern that a surface film is excessively formed on the electrode, thereby increasing the electrode thickness, and so long as the content is 0.001% by mass or more, a surface film is thoroughly formed, and the high-temperature cycle property is improved. Thus, the foregoing range is preferred. The content is more preferably 0.05% by mass or more, and still more preferably 0.1% by mass or more in the nonaqueous electrolytic solution. Its upper limit is more preferably 3% by mass or less, and still more preferably 2% by mass or less.

In the present invention, as the fluorine atom-containing or unsaturated bond-containing cyclic carbonate which is used in combination with the biphenyl group-containing carbonate compound represented by the general formula (I), one or more selected from fluorine atom-containing cyclic carbonates, such as 4-fluoro-1,3-dioxolan-2-one (FEC), trans- or cis-4,5-difluoro-1,3-dioxolan-2-one (the both will be hereunder named generically as "DFEC"), etc.; and cyclic carbonates having a carbon-carbon double bond or a carbon-carbon triple bond, such as vinylene carbonate (VC), vinyl ethylene carbonate (VEC), 4-ethynyl-1,3-dioxolan-2-one (EEC), etc., are suitably exemplified. One or more selected from 4-fluoro-1,3-dioxolan-2-one (FEC), vinylene carbonate (VC), and 4-ethynyl-1,3-dioxolan-2-one (EEC) are more preferred.

These fluorine atom-containing or unsaturated bond-containing cyclic carbonates may be used solely, or in the case where a combination of two or more of the cyclic carbonates is used, the electrochemical characteristics are more improved in a broad temperature range, and thus, such is preferred. It is preferred to use a combination of the fluorine atom-containing cyclic carbonate and the unsaturated bond-containing cyclic carbonate.

As a suitable combination of these fluorine atom-containing or unsaturated bond-containing cyclic carbonates, there are suitably exemplified FEC and DFEC; FEC and VC; FEC and VEC; FEC and EEC; DFEC and VC; DFEC and VEC; DFEC and EEC; VC and VEC; VC and EEC; VEC and EEC; FEC, DFEC and VC; FEC, DFEC and VEC; FEC, DFEC and EEC; FEC, VC and VEC; FEC, VC and EEC; FEC, VEC and EEC; DFEC, VC and VEC; DFEC, VC and EEC; DFEC, VEC and EEC; VC, VEC and EEC; and the like. Among the aforementioned combinations, combinations, such as FEC and VC; FEC and EEC; VC and VEC; VC and EEC; VEC and EEC; FEC, VC and VEC; FEC, VC and EEC; FEC, VEC and EEC; and VC, VEC and EEC are more preferred.

A content of the fluorine atom-containing cyclic carbonate is preferably 0.07% by volume or more, more preferably 1% by volume or more, and still more preferably 2% by volume or more relative to a total volume of the nonaqueous solvent, and an upper limit thereof is preferably 35% by volume or less, more preferably 25% by volume or less, still more preferably 15% by volume or less, and yet still more preferably 10% by volume or less. When the content falls within the above range, the rate of increase in electrode thickness can be preferably reduced without impairing Li ion permeability.

A content of the cyclic carbonate containing an unsaturated bond, such as a carbon-carbon double bond, a carbon-carbon triple bond, etc., is preferably 0.07% by volume or more, more preferably 0.2% by volume or more, and still more preferably 0.7% by volume or more relative to a total volume of the nonaqueous solvent, and an upper limit thereof is preferably 7% by volume or less, more preferably 4% by volume or less, and still more preferably 2.5% by volume or less. When the content falls within the above range, the rate of increase in electrode thickness can be preferably reduced without impairing Li ion permeability.

In the nonaqueous electrolytic solution of the present invention, by combining the biphenyl group-containing carbonate compound represented by the foregoing general formula (I) with a nonaqueous solvent, an electrolyte salt, and other additive as described below, there is revealed a peculiar effect such that not only the electrochemical characteristics are synergistically improved in a broad temperature range, especially at high temperatures, but also the rate of increase in electrode thickness is reduced.

[Nonaqueous Solvent]

Suitable examples of the nonaqueous solvent which is used for the nonaqueous electrolytic solution of the present invention include one or more selected from a cyclic carbonate, a linear ester, an ether, a lactone, an amide, and a sulfone. From the viewpoint of synergistically improving the electrochemical characteristics in a broad temperature range, it is preferred to contain a linear ester, it is more preferred to contain a linear carbonate, and it is the most preferred to contain both a cyclic carbonate and a linear carbonate.

The term "linear ester" as referred to in the present specification is used as a concept including a linear carbonate and a linear carboxylic acid ester.

As the cyclic carbonate, one or more selected from the aforementioned fluorine atom- or unsaturated bond-containing cyclic carbonates, ethylene carbonate (EC), propylene carbonate (PC), 1,2-butylene carbonate, and 2,3-butylene carbonate are preferred; and one or two selected from ethylene carbonate and propylene carbonate are more preferred. When the nonaqueous solvent contains ethylene carbonate and/or propylene carbonate, resistance of a surface film formed on an electrode becomes small, and hence, such is preferred. A content of ethylene carbonate and/or propylene carbonate is preferably 3% by volume or more, more preferably 5% by volume or more, and still more preferably 7% by volume or more relative to a total volume of the nonaqueous solvent, and an upper limit thereof is preferably 50% by volume or less, more preferably 45% by volume or less, and still more preferably 35% by volume or less.

These solvents may be used solely, but in the case where two or more of the solvents including the aforementioned fluorine atom-containing or unsaturated bond-containing cyclic carbonate in combination are used, the electrochemical characteristics are more improved in a broad temperature range, especially at high temperatures, and hence, such is preferred, and use of a combination of three or more thereof is especially preferred. As suitable combinations of these cyclic carbonates, EC and PC; EC and VC; PC and VC; VC and FEC; EC and FEC; PC and FEC; FEC and DFEC; EC and DFEC; PC and DFEC; VC and DFEC; VEC and DFEC; VC and EEC; EC and EEC; EC, PC and VC; EC, PC and FEC; EC, VC and FEC; EC, VC and VEC; EC, VC and EEC; EC, EEC and FEC; PC, VC and FEC; EC, VC and DFEC; PC, VC and DFEC; EC, PC, VC and FEC; EC, PC, VC and DFEC; and the like are preferred. Among the aforementioned combinations, combinations, such as EC and VC; EC and FEC; PC and FEC; EC, PC and VC; EC, PC and FEC; EC, VC and FEC; EC, VC and EEC; EC, EEC and FEC; PC, VC and FEC; EC, PC, VC and FEC; etc., are more preferred.

As the linear ester, there are suitably exemplified one or more asymmetric linear carbonates selected from

methyl ethyl carbonate (MEC), methyl propyl carbonate (MPC), methyl isopropyl carbonate (MIPC), methyl butyl carbonate, and ethyl propyl carbonate; one or more symmetric linear carbonates selected from dimethyl carbonate (DMC), diethyl carbonate (DEC), dipropyl carbonate, and dibutyl carbonate; and one or more linear carboxylic acid esters selected from pivalic acid esters, such as methyl pivalate, ethyl pivalate, propyl pivalate, etc., methyl propionate, ethyl propionate, methyl acetate, and ethyl acetate.

Among the aforementioned linear esters, linear esters having a methyl group, which are selected from dimethyl carbonate, methyl ethyl carbonate, methyl propyl carbonate, methyl isopropyl carbonate, methyl butyl carbonate, methyl propionate, methyl acetate, and ethyl acetate, are preferred, and linear carbonates having a methyl group are especially preferred.

In the case of using a linear carbonate, it is preferred to use two or more kinds thereof. Furthermore, it is more preferred that both a symmetric linear carbonate and an asymmetric linear carbonate are contained, and it is still more preferred that a content of the symmetric linear carbonate is more than that of the asymmetric linear carbonate.

Although a content of the linear ester is not particularly limited, it is preferred to use the linear ester in an amount in the range of from 60 to 90% by volume relative to a total volume of the nonaqueous solvent. When the content is 60% by volume or more, the viscosity of the nonaqueous electrolytic solution does not become excessively high, whereas it is 90% by volume or less, there is less concern that an electroconductivity of the nonaqueous electrolytic solution decreases, thereby worsening the electrochemical characteristics in a broad temperature range, especially at high temperatures, and therefore, it is preferred that the content of the linear ester falls within the foregoing range.

A proportion of the volume of the symmetric linear carbonate occupying in the linear carbonate is preferably 51% by volume or more, and more preferably 55% by volume or more. An upper limit thereof is more preferably 95% by volume or less, and still more preferably 90% by volume or less. It is especially preferred that dimethyl carbonate is contained as the symmetric linear carbonate. It is more preferred that the asymmetric linear carbonate has a methyl group, and methyl ethyl carbonate is especially preferred. The aforementioned case is preferred because the electrochemical characteristics in a much broader temperature range, especially at high temperatures are improved, and the rate of increase in electrode thickness is reduced.

As for a proportion of the cyclic carbonate and the linear ester, from the viewpoint of improving the electrochemical characteristics in a broad temperature range, especially at high temperatures, a ratio of the cyclic carbonate to the linear ester (volume ratio) is preferably 10/90 to 45/55, more preferably 15/85 to 40/60, and especially preferably 20/80 to 35/65.

As other nonaqueous solvents, there are suitably exemplified one or more selected from cyclic ethers, such as tetrahydrofuran, 2-methyltetrahydrofuran, 1,4-dioxane, etc.; linear ethers, such as 1,2-dimethoxyethane, 1,2-diethoxyethane, 1,2-dibutoxyethane, etc.; lactones, such as γ-butyrolactone, γ-valerolactone, α-angelicalactone, etc.; amides, such as dimethylformamide, etc.; and sulfones, such as sulfolane, etc.

As for the aforementioned nonaqueous solvent, in order to achieve appropriate physical properties, a mixture thereof is generally used. As a combination thereof, for example, there are suitably exemplified a combination of a cyclic carbonate and a linear carbonate, a combination of a cyclic carbonate and a linear carboxylic acid ester, a combination of a cyclic carbonate, a linear carbonate, and a lactone, a combination of a cyclic carbonate, a linear carbonate, and an ether, a combination of a cyclic carbonate, a linear carbonate, and a linear carboxylic acid ester, and the like.

In the nonaqueous electrolytic solution of the present invention, as the additive which may be used in combination with the biphenyl group-containing carbonate compound represented by the general formula (I), there is exemplified at least one selected from (a) an $SO_2$ group-containing compound, (b) a fluorinated benzene compound, (c) a phosphoric acid ester compound, (d) a carbon-carbon triple bond-containing compound, (e) a carboxylic acid anhydride, (f) an isocyanate compound, (g) a lithium-containing ionic compound, (h) a nitrile compound, (i) a benzene compound, (j) a cyclic acetal compound, and (k) a phosphazene compound. By incorporating at least one selected from the aforementioned (a) to (k) compounds, it is possible to form a mixture SEI surface film by four or more functional groups or characteristic groups and thereby further improve the effect for reducing the rate of increase in electrode thickness after high-temperature cycles.

As for (a) the $SO_2$ group-containing compound, so long as it is a compound having an "$SO_2$ group" in a molecule thereof, its kind is not particularly limited. As specific examples thereof, one or more selected from sultones, such as 1,3-propanesultone, 1,3-butanesultone, 2,4-butanesultone, 1,4-butanesultone, 1,3-propenesultone, 2,2-dioxide-1,2-oxathiolane-4-yl acetate, 5,5-dimethyl-1,2-oxathiolane-4-one 2,2-dioxide, etc.; ethylene sulfite; butane-2,3-diyl dimethanesulfonate; butane-1,4-diyl dimethanesulfonate; pentane-1,5-diyl dimethanesulfonate; methylene methanedisulfonate; divinylsulfone; and the like are suitably exemplified.

Among the aforementioned $SO_2$ group-containing compounds, one or more selected from 1,3-propanesultone, 1,4-butanesultone, 2,4-butanesultone, 2,2-dioxide-1,2-oxathiolane-4-yl acetate, 5,5-dimethyl-1,2-oxathiolane-4-one 2,2-dioxide, butane-2,3-diyl dimethanesulfonate, and divinylsulfone are more preferred.

As for (b) the fluorinated benzene compound, so long as it is a compound having a "phenyl group in which at least a part of the benzene ring is substituted with fluorine" in a molecule thereof, its kind is not particularly limited. As

specific examples thereof, one or more selected from fluorobenzene, difluorobenzene (o-, m-, or p-form), 2,4-difluoroanisole, 1-fluoro-2-cyclohexylbenzene, 1-fluoro-3-cyclohexylbenzene, 1-fluoro-4-cyclohexylbenzene, pentafluorophenyl methanesulfonate, 2-fluorophenyl methanesulfonate, 3-fluorophenyl methanesulfonate, 4-fluorophenyl methanesulfonate, 2,4-difluorophenyl methanesulfonate, 3,4-difluorophenyl methanesulfonate, 2,3,4-trifluorophenyl methanesulfonate, 2,3,5,6-tetrafluorophenyl methanesulfonate, 4-fluoro-3-trifluoromethylphenyl methanesulfonate, 4-fluoro-3-trifluoromethylphenyl methyl carbonate, and the like are suitably exemplified.

As the aforementioned fluorinated benzene compound, one or more selected from fluorobenzene, 2,4-difluoroanisole, 1-fluoro-4-cyclohexylbenzene, pentafluorophenyl methanesulfonate, 2-fluorophenyl methanesulfonate, 2,4-difluorophenyl methanesulfonate, and 4-fluoro-3-trifluoromethylphenyl methanesulfonate are more preferred.

As for (c) the phosphoric acid ester compound, so long as it is a compound having a "P(=O) group" in a molecule thereof, its kind is not particularly limited. As specific examples thereof, one or more selected from trimethyl phosphate, tributyl phosphate, trioctyl phosphate, tris(2,2,2-trifluoroethyl) phosphate, bis(2,2,2-trifluoroethyl)methyl phosphate, bis(2,2,2-trifluoroethyl)ethyl phosphate, bis(2,2,2-trifluoroethyl)2,2-difluroethyl phosphate, bis(2,2,2-trifluoroethyl)2,2,3,3-tetrafluoropropyl phosphate, bis(2,2-difluoroethyl)2,2,2-trifluoroethyl phosphate, bis(2,2,3,3-tetrafluoropropyl)2,2,2-trifluoroethyl phosphate, (2,2,2-trifluroethyl)(2,2,3,3-tetrafluoropropyl)methyl phosphate, tris(1,1,1,3,3,3-hexafluoropropan-2-yl) phosphate, methyl methylenebisphosphonate, ethyl methylenebisphosphonate, methyl ethylenebisphosphonate, ethyl ethylenebisphosphonate, methyl butylenebisphosphonate, ethyl butylenebisphosphonate, methyl 2-(dimethylphosphoryl) acetate, ethyl 2-(dimethylphosphoryl) acetate, methyl 2-(diethylphosphoryl) acetate, ethyl 2-(diethylphosphoryl) acetate, 2-propynyl 2-(dimethylphosphoryl) acetate, 2-propynyl 2-(diethylphosphoryl) acetate, methyl 2-(dimethoxyphosphoryl) acetate, ethyl 2-(dimethoxyphosphoryl) acetate, methyl 2-(diethoxyphosphoryl) acetate, ethyl 2-(diethoxyphosphoryl) acetate, 2-propynyl 2-(dimethoxyphosphoryl) acetate, 2-propynyl 2-(diethoxyphosphoryl) acetate, methyl pyrophosphate, ethyl pyrophosphate, and the like are suitably exemplified.

Among the aforementioned phosphoric acid ester compounds, tris(2,2,2-trifluoroethyl) phosphate, tris(1,1,1,3,3,3-hexafluoropropan-2-yl) phosphate, methyl 2-(dimethoxyphosphoryl) acetate, ethyl 2-(dimethoxyphosphoryl) acetate, methyl 2-(diethoxyphosphoryl) acetate, ethyl 2-(diethoxyphosphoryl) acetate, 2-propynyl 2-(dimethoxyphosphoryl) acetate, and 2-propynyl 2-(diethoxyphosphoryl) acetate are preferred; and one or more selected from tris(2,2,2-trifluoroethyl) phosphate, tris(1,1,1,3,3,3-hexafluoropropan-2-yl) phosphate, ethyl 2-(diethoxyphosphoryl) acetate, and 2-propynyl 2-(diethoxyphosphoryl) acetate are more preferred.

As for (d) the carbon-carbon triple bond-containing compound, so long as it is a compound having a "carbon-carbon triple bond" in a molecule thereof, its kind is not particularly limited. As specific examples thereof, one or more selected from 2-propynyl methyl carbonate, 2-propynyl acetate, 2-propynyl formate, 2-propynyl methacrylate, 2-propynyl methanesulfonate, 2-propynyl vinylsulfonate, 2-propynyl 2-(methanesulfonyloxy)propionate, di(2-propynyl) oxalate, methyl 2-propynyl oxalate, ethyl 2-propynyl oxalate, di(2-propynyl) glutarate, 2-butyne-1,4-diyl dimethanesulfonate, 2-butyne-1,4-diyl diformate, 2,4-hexadiyne-1,6-diyl dimethanesulfonate, and the like are suitably exemplified.

Among the aforementioned triple bond-containing compounds, one or more selected from 2-propynyl methyl carbonate, 2-propynyl methacrylate, 2-propynyl methanesulfonate, 2-propynyl vinylsulfonate, 2-propynyl 2-(methanesulfonyloxy)propionate, di(2-propynyl) oxalate, methyl 2-propynyl oxalate, ethyl 2-propynyl oxalate, 2-butyne-1,4-diyl dimethanesulfonate, and 2-butyne-1,4-diyl diformate are preferred; and one or more selected from 2-propynyl methyl carbonate, 2-propynyl methanesulfonate, 2-propynyl vinylsulfonate, 2-propynyl 2-(methanesulfonyloxy)propionate, di(2-propynyl) oxalate, 2-butyne-1,4-diyl dimethanesulfonate, and 2-butyne-1,4-diyl diformate are more preferred.

As for (e) the carboxylic acid anhydride, so long as it is a compound having a "C(=O)-O-C(=O) group" in a molecule thereof, its kind is not particularly limited. As specific examples thereof, one or more selected from linear carboxylic acid anhydrides, such as acetic anhydride, propionic anhydride, etc.; cyclic acid anhydrides, such as succinic anhydride, maleic anhydride, allyl succinic anhydride, glutaric anhydride, itaconic anhydride, 3-sulfo-propionic anhydride, etc.; and the like are suitably exemplified.

Among the aforementioned carboxylic acid anhydrides, one or more selected from succinic anhydride, maleic anhydride, and allyl succinic anhydride are preferred; and one or two selected from succinic anhydride and allyl succinic anhydride are more preferred.

As for (f) the isocyanate compound, so long as it is a compound having an "N=C=O group", its kind is not particularly limited. As specific examples thereof, one or more selected from methyl isocyanate, ethyl isocyanate, butyl isocyanate, phenyl isocyanate, tetramethylene diisocyanate, hexamethylene diisocyanate, octamethylene diisocyanate, 1,4-phenylene diisocyanate, 2-isocyanatoethyl acrylate, 2-isocyanatoethyl methacrylate, and the like are suitably exemplified.

Among the aforementioned isocyanate compounds, one or more selected from hexamethylene diisocyanate, octamethylene diisocyanate, 2-isocyanatoethyl acrylate, and 2-isocyanatoethyl methacrylate are preferred; and one or more selected from hexamethylene diisocyanate, 2-isocyanatoethyl acrylate, and 2-isocyanatoethyl methacrylate are more preferred.

As for (g) the lithium-containing ionic compound, so long as it is a compound having "lithium" as a cation species,

its kind is not particularly limited. As specific examples thereof, one or more selected from lithium difluorophosphate, lithium fluorophosphate, lithium fluorosulfonate, lithium difluorobis[oxalate-O,O']phosphate (LiPFO), lithium tetrafluoro[oxalate-O,O']phosphate, lithium bis[oxalate-O,O']borate (LiBOB), lithium difluoro[oxalate-O,O']borate, lithium methyl sulfate, lithium ethyl sulfate, lithium propyl sulfate, and the like are suitably exemplified.

Among the aforementioned lithium-containing ionic compounds, one or more selected from lithium difluorophosphate, lithium fluorosulfonate, lithium difluorobis[oxalate-O,O']phosphate (LiPFO), lithium tetrafluoro[oxalate-O,O']phosphate, lithium bis[oxalate-O,O']borate (LiBOB), lithium difluoro[oxalate-O,O']borate, lithium methyl sulfate, lithium ethyl sulfate, and lithium propyl sulfate are more preferred.

As for (h) the nitrile compound, so long as it is a compound having a "nitrile group", its kind is not particularly limited. As specific examples thereof, one or more selected from acetonitrile, propionitrile, succinonitrile, glutaronitrile, adiponitrile, pimelonitrile, suberonitrile, and sebaconitrile are suitably exemplified.

Among the aforementioned nitrile compounds, one or more selected from succinonitrile, glutaronitrile, adiponitrile, and pimelonitrile are more preferred.

As for (i) the benzene compound, so long as it is a compound having a "phenyl group" in a molecule thereof, its kind is not particularly limited. As specific examples thereof, one or more selected from aromatic compounds having a branched alkyl group, such as cyclohexylbenzene, tert-butylbenzene, tert-amylbenzene, etc.; biphenyl; terphenyl (o-, m-, or p-form); diphenyl ether; anisole; partial hydrides of terphenyl (e.g., 1,2-dicyclohexylbenzene, 2-phenylbicyclohexyl, 1,2-diphenylcyclohexane, or o-cyclohexylbiphenyl); and phenyl carbonate compounds, such as methyl phenyl carbonate, ethyl phenyl carbonate, diphenyl carbonate, etc., are suitably exemplified.

Among the aforementioned benzene compounds, one or more selected from cyclohexylbenzene, tert-butylbenzene, tert-amylbenzene, biphenyl, terphenyl (o-, m-, or p-form), methyl phenyl carbonate, ethyl phenyl carbonate, and diphenyl carbonate are more preferred; and cyclohexylbenzene, tert-amylbenzene, biphenyl, o-terphenyl, methyl phenyl carbonate, ethyl phenyl carbonate, and diphenyl carbonate are especially preferred.

As for (j) the cyclic acetal compound, so long as it is a compound having an "acetal group" in a molecule thereof, its kind is not particularly limited. As specific examples thereof, one or more selected from 1,3-dioxolane, 1,3-dioxane, 1,3,5-trioxane, and the like are suitably exemplified.

Among the aforementioned cyclic acetal compounds, 1,3-dioxolane and 1,3-dioxane are preferred, and 1,3-dioxane is more preferred.

As for (k) the phosphazene compound, so long as it is a compound having an "N=P-N group" in a molecule thereof, its kind is not particularly limited. As specific examples thereof, one or more selected from methoxypentafluorocyclotriphosphazene, ethoxypentafluorocyclotriphosphazene, phenoxypentafluorocyclotriphosphazene, ethoxyheptafluorocyclotetraphosphazene, and the like are suitably exemplified.

Among the aforementioned phosphazene compounds, cyclic phosphazene compounds, such as methoxypentafluorocyclotriphosphazene, ethoxypentafluorocyclotriphosphazene, phenoxypentafluorocyclotriphosphazene, etc., are preferred; and one or two selected from methoxypentafluorocyclotriphosphazene and ethoxypentafluorocyclotriphosphazene are more preferred.

A content of each of the aforementioned (a) $SO_2$ group-containing compound, (b) fluorinated benzene compound, (c) phosphoric acid ester compound, (d) carbon-carbon triple bond-containing compound, (e) carboxylic acid anhydride, (f) isocyanate compound, (g) lithium-containing ionic compound, (h) nitrile compound, (i) benzene compound, (j) cyclic acetal compound, and (k) phosphazene compound is preferably 0.001 to 5% by mass in the nonaqueous electrolytic solution. In this range, not only a surface film is thoroughly formed without becoming excessively thick, and the effect for improving electrochemical characteristics in a broad temperature range, especially at high temperatures is increased, but also the rate of increase in electrode thickness can be much more reduced. The content is more preferably 0.01% by mass or more, and still more preferably 0.1% by mass or more in the nonaqueous electrolytic solution. Its upper limit is more preferably 3.5% by mass or less, and still more preferably 2.5% by mass or less.

As for the compound which is used in combination with the compound represented by the foregoing general formula (I), it is preferred to use two or more of the compounds in combination. Among the combinations, it is more preferred to use (g) the lithium-containing ionic compound in combination with at least one selected from (a) the $SO_2$ group-containing compound, (b) the fluorinated benzene compound, (c) the phosphoric acid ester compound, (d) the carbon-carbon triple bond-containing compound, (e) the carboxylic acid anhydride, (f) the isocyanate compound, (h) the nitrile compound, (i) the benzene compound, (j) the cyclic acetal compound, and (k) the phosphazene compound; and it is still more preferred to use (g) the lithium-containing ionic compound in combination with at least one selected from (a) the $SO_2$ group-containing compound, (b) the fluorinated benzene compound, (c) the phosphoric acid ester compound, (d) the carbon-carbon triple bond-containing compound, (f) the isocyanate compound, (h) the nitrile compound, and (i) the benzene compound.

[Electrolyte Salt]

As the electrolyte salt which is used in the present invention, lithium salts are suitably exemplified.

As the lithium salt, one or more of inorganic lithium salts, such as $LiPF_6$, $LiBF_4$, $LiClO_4$, $LiN(SO_2F)_2$, etc.; lithium salts containing a linear fluorinated alkyl group, such as $LiN(SO_2CF_3)_2$, $LiN(SO_2C_2Fs)_2$, $LiCF_3SO_3$, $LiC(SO_2CF_3)_3$, $LiPF_4(CF_3)_2$, $LiPF_3(C_2F_5)_3$, $LiPF_3(CF_3)_3$, $LiPF_3(iso\text{-}C_3F_7)_3$, $LiPF_5(iso\text{-}C_3F_7)$, etc.; and lithium salts having a cyclic fluorinated alkylene chain, such as $(CF_2)_2(SO_2)_2NLi$, $(CF_2)_3(SO_2)_2NLi$, etc., may be used.

Of those, one or more selected from $LiPF_6$, $LiBF_4$, $LiN(SO_2F)_2$, $LiN(SO_2CF_3)_2$, and $LiN(SO_2C_2F_5)_2$ are preferred; one or more selected from $LiPF_6$, $LiBF_4$, $LiN(SO_2CF_3)_2$, and $LiN(SO_2F)_2$ are more preferred; and it is the most preferred to use $LiPF_6$.

In general, a concentration of the lithium salt is preferably 0.3 M or more, more preferably 0.7 M or more, and still more preferably 1.1 M or more relative to the nonaqueous solvent. Its upper limit is preferably 2.5 M or less, more preferably 2.0 M or less, and still more preferably 1.6 M or less.

As a suitable combination of these lithium salts, a combination including $LiPF_6$ and one or more selected from $LiN(SO_2F)_2$ is more preferred. When a proportion of the lithium salt other than $LiPF_6$ occupying in the nonaqueous solvent is 0.001 M or more, the effect for improving electrochemical characteristics at high temperatures is liable to be exhibited, whereas when it is 0.5 M or less, there is less concern that the effect for improving electrochemical characteristics at high temperature is worsened, and hence, such is preferred. The proportion of the lithium salt other than $LiPF_6$ is preferably 0.01 M or more, especially preferably 0.03 M or more, and most preferably 0.04 M or more. Its upper limit is preferably 0.4 M or less, and especially preferably 0.2 M or less.

In the case where $LiPF_6$ is contained in the nonaqueous electrolytic solution, when a ratio of the biphenyl group-containing carbonate compound represented by the general formula (I) to $LiPF_6$ in terms of a molar concentration is 0.0005 or more, the effect for improving electrochemical characteristics at high temperatures is liable to be exhibited, whereas when it is 0.3 or less, there is less concern that the effect for improving electrochemical characteristics at high temperatures is worsened, and hence, such is preferred. Its lower limit is more preferably 0.001 or more, and still more preferably 0.005 or more. Its upper limit is more preferably 0.2 or less, and still more preferably 0.1 or less.

[Production of Nonaqueous Electrolytic Solution]

The nonaqueous electrolytic solution of the present invention can be, for example, obtained by mixing the aforementioned nonaqueous solvent and adding the biphenyl group-containing carbonate compound represented by the foregoing general formula (I) to the aforementioned electrolyte salt and the nonaqueous electrolytic solution.

At this time, the nonaqueous solvent used and the compound added to the nonaqueous electrolytic solution are preferably purified previously to reduce as much as possible the content of impurities, in such an extent that the productivity is not extremely deteriorated.

The nonaqueous electrolytic solution of the present invention may be used in first to fourth energy storage devices described below, in which the nonaqueous electrolyte may be used not only in the form of a liquid but also in the form of a gel. Furthermore, the nonaqueous electrolytic solution of the present invention may also be used for a solid polymer electrolyte. Above all, the nonaqueous electrolytic solution is preferably used in the first energy storage device using a lithium salt as the electrolyte salt (i.e., for a lithium battery) or in the fourth energy storage device (i.e., for a lithium ion capacitor), more preferably used in a lithium battery, and still more preferably used in a lithium secondary battery.

[First Energy Storage Device (Lithium Battery)]

The lithium battery as referred to in the present specification is a generic name for a lithium primary battery and a lithium secondary battery. In the present specification, the term, lithium secondary battery, is used as a concept also including a so-called lithium ion secondary battery. The lithium battery of the present invention contains a positive electrode, a negative electrode, and the aforementioned nonaqueous electrolytic solution having an electrolyte salt dissolved in a nonaqueous solvent. Other constitutional members used than the nonaqueous electrolytic solution, such as the positive electrode, the negative electrode, etc., may be used without being particularly limited.

For example, as the positive electrode active material for lithium secondary batteries, usable is a complex metal oxide containing lithium and one or more selected from cobalt, manganese, and nickel. These positive electrode active materials may be used solely or in combination of two or more kinds thereof.

As the lithium complex metal oxides, for example, one or more selected from $LiCoO_2$, $LiMn_2O_4$, $LiNiO_2$, $LiCo_{1-x}Ni_xO_2$ ($0.01 < x < 1$), $LiCo_{1/3}Ni_{1/3}Mn_{1/3}O_2$, $LiNi_{1/2}Mn_{3/2}O_4$, and $LiCo_{0.98}Mg_{0.02}O_2$ are exemplified. These materials may be used as a combination, such as a combination of $LiCoO_2$ and $LiMn_2O_4$, a combination of $LiCoO_2$ and $LiNiO_2$, and a combination of $LiMn_2O_4$ and $LiNiO_2$.

For improving the safety on overcharging and the cycle property, and for enabling the use at a charge potential of 4.3 V or more, a part of the lithium complex metal oxide may be substituted with other elements. For example, a part of cobalt, manganese, or nickel may be substituted with at least one or more elements of Sn, Mg, Fe, Ti, Al, Zr, Cr, V, Ga, Zn, Cu, Bi, Mo, La, etc.; or a part of O may be substituted with S or F; or the oxide may be coated with a compound containing any of such other elements.

Of those, preferred are lithium complex metal oxides, such as $LiCoO_2$, $LiMn_2O_4$, and $LiNiO_2$, with which the charge potential of the positive electrode in a fully-charged state may be used at 4.3 V or more based on Li: and more preferred are lithium complex metal oxides, such as $LiCo_{1-x}M_xO_2$ (wherein M is one or more elements selected from Sn, Mg, Fe, Ti, Al, Zr, Cr, V, Ga, Zn, and Cu; and $0.001 \leq x \leq 0.05$), $LiNi_{1/3}Mn_{1/3}Co_{1/3}O_2$, $LiNi_{1/2}Mn_{3/2}O_4$, and a solid solution of $Li_2MnO_3$ and $LiMO_2$ (wherein M is a transition metal, such as Co, Ni, Mn, Fe, etc.), that may be used at 4.4 V or more. The use of the lithium complex metal oxide capable of acting at a high charge voltage is liable to worsen the electrochemical characteristics particularly on using in a broad temperature range due to the reaction with the electrolytic solution on charging, but in the lithium secondary battery according to the present invention, the electrochemical characteristics can be prevented from worsening. In particular, in the case of a positive electrode containing Mn, there is a tendency that the resistance of the battery is liable to increase with elution of an Mn ion from the positive electrode, so that there is a tendency that when used in a broad temperature range, the electrochemical characteristics are liable to be worsened. However, in the lithium secondary battery according to the present invention, the worsening of these electrochemical characteristics can be suppressed, and hence, such is preferred.

Furthermore, a lithium-containing olivine-type phosphate may also be used as the positive electrode active material. Especially preferred are lithium-containing olivine-type phosphates containing one or more selected from iron, cobalt, nickel, and manganese. Specific examples thereof include one or more selected from $LiFePO_4$, $LiCoPO_4$, $LiNiPO_4$, $LiMnPO_4$, and $LiFe_{1-x}Mn_xPO_4$ ($0.1 < x < 0.9$). Of those, $LiFePO_4$ and $LiMnPO_4$ are more preferred, and $LiFePO_4$ is still more preferred.

These lithium-containing olivine-type phosphates may be partly substituted with any other element; and for example, a part of iron, cobalt, nickel, or manganese therein may be substituted with one or more elements selected from Co, Mn, Ni, Mg, Al, B, Ti, V, Nb, Cu, Zn, Mo, Ca, Sr, W, and Zr; or the phosphates may also be coated with a compound containing any of these other elements or with a carbon material. Of those, $LiFePO_4$ and $LiMnPO_4$ are preferred. The lithium-containing olivine-type phosphate may be used, for example, in combination with the aforementioned positive electrode active material.

The lithium-containing olivine-type phosphate forms a stable phosphoric acid skeleton ($PO_4$) structure and is excellent in heat stability on charging, so that the rate of increase in electrode thickness after high-temperature cycles can be more reduced.

For the positive electrode for lithium primary batteries, there are suitably exemplified oxides or chalcogen compounds of one or more metal elements, such as CuO, $Cu_2O$, $Ag_2O$, $Ag_2CrO_4$, CuS, $CuSO_4$, $TiO_2$, TiS2, $SiO_2$, SnO, $V_2O_5$, $V_6O_{12}$, VOx, $Nb_2O_5$, $Bi_2O_3$, $Bi_2Pb_2O_5$, $Sb_2O_3$, $CrO_3$, $Cr_2O_3$, $MoO_3$, $WO_3$, $SeO_2$, $MnO_2$, $Mn_2O_3$, $Fe_2O_3$, FeO, $Fe_3O_4$, $Ni_2O_3$, NiO, $CoO_3$, CoO, etc.; sulfur compounds, such as $SO_2$, $SOCl_2$, etc.; and carbon fluorides (graphite fluoride) represented by a general formula $(CF_x)_n$. Above all, $MnO_2$, $V_2O_5$, graphite fluoride, and the like are preferred.

In the case where a pH of a supernatant at the time of dispersing 10 g of the aforementioned positive electrode active material in 100 mL of distilled water is 10.0 to 12.5, the effect for improving electrochemical characteristics in a much broader temperature range is liable to be obtained, and hence, such is preferred. The case where the pH is 10.5 to 12.0 is more preferred.

In the case where Ni is contained as the element in the positive electrode, since there is a tendency that impurities, such as LiOH, etc., in the positive electrode active material increase, the effect for improving electrochemical characteristics in a much broader temperature range is liable to be obtained, and hence, such is preferred. The case where an atomic concentration of Ni in the positive electrode active material is 5 to 25 atomic% is more preferred, and the case where it is 8 to 21 atomic% is especially preferred.

An electroconductive agent of the positive electrode is not particularly limited so long as it is an electron-conductive material that does not undergo a chemical change. Examples thereof include graphites, such as natural graphite (e.g., flaky graphite, etc.), artificial graphite, etc.; one or more carbon blacks selected from acetylene black, Ketjen black, channel black, furnace black, lamp black, and thermal black; and the like. Graphite and carbon black may be properly mixed and used. An addition amount of the electroconductive agent to the positive electrode mixture is preferably from 1 to 10% by mass, and especially preferably from 2 to 5% by mass.

The positive electrode may be produced by mixing the aforementioned positive electrode active material with an electroconductive agent, such as acetylene black, carbon black, etc., and a binder, such as polytetrafluoroethylene (PTFE), polyvinylidene fluoride (PVDF), a copolymer of styrene and butadiene (SBR), a copolymer of acrylonitrile and butadiene (NBR), carboxymethyl cellulose (CMC), an ethylene-propylene-diene terpolymer, etc., adding a high-boiling point solvent, such as 1-methyl-2-pyrrolidone, etc., thereto, followed by kneading to prepare a positive electrode mixture, applying this positive electrode mixture onto a collector, such as an aluminum foil, a stainless steel-made lath plate, etc.,

and drying and shaping the resultant under pressure, followed by a heat treatment in vacuum at a temperature of about 50°C to 250°C for about 2 hours.

A density of a portion of the positive electrode except for the collector is generally 1.5 g/cm$^3$ or more, and for the purpose of further increasing the capacity of the battery, the density is preferably 2 g/cm$^3$ or more, more preferably 3 g/cm$^3$ or more, and still more preferably 3.6 g/cm$^3$ or more. An upper limit thereof is preferably 4 g/cm$^3$ or less.

As the negative electrode active material for lithium secondary batteries, one or more selected from a lithium metal, lithium alloys, carbon materials capable of absorbing and releasing lithium [e.g., graphitizable carbon, non-graphitizable carbon having a lattice (002) spacing of 0.37 nm or more, graphite having a lattice (002) spacing of 0.34 nm or less, etc.], tin (elemental substance), tin compounds, silicon (elemental substance), silicon compounds, lithium titanate compounds, such as $Li_4Ti_5O_{12}$, etc., and the like may be used solely or in combination.

Of those, in absorbing and releasing ability of a lithium ion, it is more preferred to use a high-crystalline carbon material, such as artificial graphite, natural graphite, etc.; and it is still more preferred to use a carbon material having a graphite-type crystal structure in which a lattice (002) spacing ($d_{002}$) is 0.340 nm (nanometers) or less, especially 0.335 to 0.337 nm. In particular, it is preferred to use an artificial graphite particle having a bulky structure in which plural flat graphite fine particles are mutually gathered or bound in non-parallel, or a graphite particle prepared by subjecting a flaky natural graphite particle to a spheroidizing treatment by repeatedly giving a mechanical action, such as compression force, frictional force, shear force, etc.

When a ratio [I(110)/I(004)] of a peak intensity I(110) of the (110) plane to a peak intensity I(004) of the (004) plane of the graphite crystal, which is obtained from the X-ray diffraction measurement of a negative electrode sheet at the time of shaping under pressure of a portion of the negative electrode except for the collector in a density of 1.5 g/cm$^3$ or more, is 0.01 or more, the electrochemical characteristics in a much broader temperature range are improved, and hence, such is preferred. The peak intensity ratio [I(110)/I(004)] is more preferably 0.05 or more, and still more preferably 0.1 or more. When excessively treated, there may be the case where the crystallinity is worsened, and the discharge capacity of the battery is worsened, and therefore, an upper limit of the peak intensity ratio [I(110)/I(004)] is preferably 0.5 or less, and more preferably 0.3 or less.

In addition, when the high-crystalline carbon material (core material) is coated with a carbon material that is more low-crystalline than the core material, the electrochemical characteristics in a broad temperature range become much more favorable, and hence, such is preferred. The crystallinity of the carbon material of the coating may be confirmed by TEM.

When the high-crystalline carbon material is used, there is a tendency that it reacts with the nonaqueous electrolytic solution on charging, thereby worsening the electrochemical characteristics at low temperatures or high temperatures due to an increase of the interfacial resistance; however, in the lithium secondary battery according to the present invention, the electrochemical characteristics in a broad temperature range become favorable.

As the metal compound capable of absorbing and releasing lithium, serving as a negative electrode active material, there are preferably exemplified compounds containing at least one metal element, such as Si, Ge, Sn, Pb, P, Sb, Bi, Al, Ga, In, Ti, Mn, Fe, Co, Ni, Cu, Zn, Ag, Mg, Sr, Ba, etc. These metal compounds may be in any form including an elemental substance, an alloy, an oxide, a nitride, a sulfide, a boride, an alloy with lithium, and the like; however, any of an elemental substance, an alloy, an oxide, and an alloy with lithium is preferred because the battery capacity may be increased. Above all, more preferred are those containing at least one element selected from Si, Ge, and Sn, and still more preferred are those containing at least one element selected from Si and Sn, as capable of increasing the battery capacity.

The negative electrode may be produced in such a manner that the same electroconductive agent, binder, and high-boiling point solvent as in the production of the aforementioned positive electrode are used and kneaded to prepare a negative electrode mixture, and this negative electrode mixture is then applied onto a collector, such as a copper foil, etc., dried, shaped under pressure, and then heat-treated in vacuum at a temperature of about 50 to 250°C for about 2 hours.

A density of a portion of the negative electrode except for the collector is generally 1.1 g/cm$^3$ or more, and for the purpose of further increasing the capacity of the battery, the density is preferably 1.5 g/cm$^3$ or more. Its upper limit is preferably 2 g/cm$^3$ or less.

Examples of the negative electrode active material for lithium primary batteries include a lithium metal and a lithium alloy.

The structure of the lithium battery is not particularly limited, and a coin-type battery, a cylinder-type battery, a prismatic battery, a laminate-type battery, or the like, each having a single-layered or multi-layered separator, may be applied thereto.

Although the separator for the battery is not particularly limited, a single-layered or laminated micro-porous film of a polyolefin, such as polypropylene, polyethylene, etc., as well as a woven fabric, a nonwoven fabric, etc., may be used.

The lithium secondary battery of the present invention has excellent electrochemical characteristics in a broad temperature range even when the final charging voltage is 4.2 V or more, and particularly 4.3 V or more, and furthermore,

the characteristics thereof are still good even at 4.4 V or more. Although the final discharging voltage may be generally 2.8 V or more, and further 2.5 V or more, the final discharging voltage of the lithium secondary battery of the present invention may be 2.0 V or more. Although a current value is not specifically limited, in general, the battery is used within the range of from 0.1 to 30 C. The lithium battery of the present invention may be charged/discharged at -40 to 100°C, and preferably at -10 to 80°C.

In the present invention, as a countermeasure against an increase in the internal pressure of the lithium battery, such a method may be employed that a safety valve is provided in the battery cap, and a cutout is provided in the battery component, such as a battery can, a gasket, etc. In addition, as a safety countermeasure for preventing overcharging, a current cut-off mechanism capable of detecting an internal pressure of the battery to cut off the current may be provided in a battery cap.

[Second Energy Storage Device (Electric Double Layer Capacitor)]

The second energy storage device of the present invention is an energy storage device that contains the nonaqueous electrolytic solution of the present invention and stores energy by utilizing the electric double layer capacitance in the interface between the electrolytic solution and the electrode therein. One example of the present invention is an electric double layer capacitor. The most typical electrode active material to be used in this energy storage device is active carbon. The double-layer capacitance increases almost in proportion to the surface area.

[Third Energy Storage Device]

The third energy storage device of the present invention is an energy storage device that contains the nonaqueous electrolytic solution of the present invention and stores energy by utilizing the doping/dedoping reaction of the electrode therein. As the electrode active material for use in this energy storage device, there may be mentioned metal oxides, such as ruthenium oxide, iridium oxide, tungsten oxide, molybdenum oxide, copper oxide, etc.; and $\pi$-conjugated polymers, such as polyacene, polythiophene derivatives, etc. The capacitor that uses such an electrode active material enables energy storage along with the doping/dedoping reaction at the electrode therein.

[Fourth Energy Storage Device (Lithium Ion Capacitor)]

The fourth energy storage device of the present invention is an energy storage device that contains the nonaqueous electrolytic solution of the present invention and stores energy by utilizing intercalation of a lithium ion into a carbon material, such as graphite, etc., servicing as the negative electrode. This energy storage device is referred to as a lithium ion capacitor (LIC). Examples of the positive electrode include one utilizing an electric double layer between an active carbon electrode and an electrolytic solution, one utilizing doping/dedoping reaction of a $\pi$-conjugated polymer electrode, and the like. The electrolytic solution contains at least a lithium salt, such as $LiPF_6$, etc.

EXAMPLES

Synthesis Examples of the biphenyl group-containing carbonate compounds to be used in the present invention are hereunder described, but it should not be construed that the present invention is limited to these Synthesis Examples.

Synthesis Example 1 [Synthesis of 2-Benzyl-6-phenylphenyl Phenyl Carbonate (Compound A63)]

To 5.00 g (19.2 mmoles) of 2-benzyl-6-phenylphenol, 1.99 g (21.1 mmoles) of methyl chloroformate, and 20 mL of ethyl acetate, 2.14 g (21.1 mmoles) of triethylamine was added dropwise at an inner temperature of 2°C to 10°C over 10 minutes, followed by stirring at room temperature for one hour. Thereafter, a reaction solution was washed with water and subjected to liquid separation, and an organic layer was concentrated. The resulting residue was purified by means of silica gel column chromatography (eluted with ethylene acetate/hexane = 1/20), thereby obtaining 5.91 g (97%) of target 2-benzyl-6-phenylphenyl phenyl carbonate.

The obtained 2-benzyl-6-phenylphenyl phenyl carbonate was subjected to $^1$H-NMR and CI-MS measurements to confirm its structure. The results are shown below.

(1) $^1$H-NMR (400 MHz, $CDCl_3$): $\delta$ = 7.45 to 7.14 (m, 13H), 3.99 (s, 2H), 3.51 (s, 3H)
(2) Melting point: 86 to 88°C
(3) Mass analysis: MS (CI) m/z = 319 $[M+1]^+$

Synthesis Example 2 [Synthesis of Phenyl 2-Phenylphenyl Carbonate (Compound B27)]

To 10.00 g (58.8 mmoles) of 2-phenylphenol, 9.66 g (61.7 mmoles) of phenyl chloroformate, and 50 mL of ethyl acetate, 6.24 g (61.7 mmoles) of triethylamine was added dropwise at an inner temperature of 2°C to 10°C over 20 minutes, followed by stirring at room temperature for one hour. Thereafter, a reaction solution was washed with water and subjected to liquid separation, and an organic layer was concentrated. To the resulting residue, 40 mL of dimethyl carbonate was added, followed by purification by crystallization, thereby obtaining 4.25 g (25%) of target phenyl 2-phenylphenyl carbonate.

The obtained phenyl 2-phenylphenyl carbonate was subjected to $^1$H-NMR and CI-MS measurements to confirm its structure. The results are shown below.

(1) $^1$H-NMR (400 MHz, $CDCl_3$) : $\delta$ = 7.53 to 6.87 (m, 14H)
(2) Melting point: 99 to 100°C
(3) Mass analysis: MS (CI) m/z = 291 $[M+1]^+$

Synthesis Example 3 [Synthesis of 2,6-Diphenylphenyl Methyl Carbonate (Compound A1)]

To 5.00 g (20.3 mmoles) of 2,6-diphenylphenol, 2.12 g (22.4 mmoles) of methyl chloroformate, and 20 mL of ethyl acetate, 2.27 g (22.4 mmoles) of triethylamine was added dropwise at an inner temperature of 2°C to 10°C over 10 minutes, followed by stirring at room temperature for one hour. Thereafter, a reaction solution was washed with water and subjected to liquid separation, and an organic layer was concentrated. To the resulting residue, 10 mL of dimethyl carbonate was added, followed by purification by crystallization, thereby obtaining 2.25 g (36%) of target 2,6-diphenylphenyl methyl carbonate.

The obtained 2,6-diphenylphenyl methyl carbonate was subjected to $^1$H-NMR and CI-MS measurements to confirm its structure. The results are shown below.

(1) $^1$H-NMR (400 MHz, $CDCl_3$): $\delta$ = 7.60 to 7.33 (m, 13H)
(2) Melting point: 110 to 111°C
(3) Mass analysis: MS (CI) m/z = 305 $[M+1]^+$

Examples of electrolytic solutions using the biphenyl group-containing carbonate of the present invention are hereunder described, but it should not be construed that the present invention is limited to these Examples.

Examples 1-1 to 1-18 and 2-1 to 2-20, and Comparative Example 1-1

[Production of Lithium Ion Secondary Battery]

94% by mass of $LiNi_{1/3}Mn_{1/3}Co_{1/3}O_2$ and 3% by mass of acetylene black (electroconductive agent) were mixed and then added to and mixed with a solution which had been prepared by dissolving 3% by mass of polyvinylidene fluoride (binder) in 1-methyl-2-pyrrolidone in advance, thereby preparing a positive electrode mixture paste. This positive electrode mixture paste was applied onto one surface of an aluminum foil (collector), dried, and treated under pressure, followed by cutting into a predetermined size, thereby producing a strip-shaped positive electrode sheet. A density of a portion of the positive electrode except for the collector was 3.6 g/cm$^3$.

95% by mass of artificial graphite ($d_{002}$ = 0.335 nm, negative electrode active material) was added to and mixed with a solution which had been prepared by dissolving 5% by mass of polyvinylidene fluoride (binder) in 1-methyl-2-pyrrolidone in advance, thereby preparing a negative electrode mixture paste. This negative electrode mixture paste was applied onto one surface of a copper foil (collector), dried, and treated under pressure, followed by cutting out into a predetermined size, thereby producing a negative electrode sheet. A density of a portion of the negative electrode except for the collector was 1.5 g/cm$^3$. This electrode sheet was analyzed by means of X-ray diffraction, and as a result, a ratio [I(110)/I(004)] of a peak intensity I(110) of the (110) plane to a peak intensity I(004) of the (004) plane of the graphite crystal was found to be 0.1.

The above-obtained positive electrode sheet, a micro-porous polyethylene film-made separator, and the above-obtained negative electrode sheet were laminated in this order, and a nonaqueous electrolytic solution having each of compositions shown in Tables 1 to 5 was added thereto, thereby producing laminate-type batteries.

[Evaluation of Low-Temperature Property after High-Temperature Charging Storage]

[Evaluation of High-Temperature Cycle Property]

In a thermostatic chamber at 65°C, each of the batteries produced by the aforementioned method was treated by repeating a cycle of charging up to a final voltage of 4.3 V with a constant current of 1 C and under a constant voltage for 3 hours and subsequently discharging down to a discharge voltage of 3.0 V with a constant current of 1 C, until it reached 100 cycles. Then, a discharge capacity retention rate after the cycles was determined according to the following equation.

$$\text{Discharge capacity retention rate (\%)} = \text{(Discharge capacity after 100th cycle)/(Discharge capacity after 1st cycle)} \times 100$$

<Initial Negative Electrode Thickness>

The battery having been subjected to one cycle by the aforementioned method was disassembled to measure an initial negative electrode thickness.

<Negative Electrode Thickness after Cycles>

The battery having been subjected to 100 cycles by the aforementioned method was disassembled to measure a negative electrode thickness after high-temperature cycles.

<Rate of Increase in Negative Electrode Thickness>

A value of increase in negative electrode thickness was determined according to the following equation.

$$\text{Value of increase in negative electrode thickness} = \text{(Negative electrode thickness after cycles)} - \text{(Initial negative electrode thickness)}$$

The rate of increase in negative electrode thickness (%) was determined on the basis that the value of increase in negative electrode thickness of Comparative Example 1-1 was defined as 100%.

Table 1

| | Composition of electrolyte salt Composition of nonaqueous electrolytic solution (Volume ratio of solvent) | Compound of the general formula (I) (% by mass) | Discharge capacity retention rate after 100 cycles at 65°C (%) | Rate of increase in negative electrode thickness after 100 cycles at 65°C (%) |
|---|---|---|---|---|
| Example 1-1 | 1.1M $LiPF_6$ EC/MEC (30/70) | 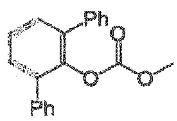 (1) | 67 | 59 |
| Example 1-2 | 1.1M $LiPF_6$ EC/DMC/MEC (30/60/10) | 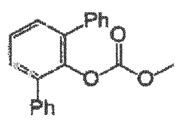 (1) | 68 | 57 |

(continued)

| | Composition of electrolyte salt Composition of nonaqueous electrolytic solution (Volume ratio of solvent) | Compound of the general formula (I) (% by mass) | Discharge capacity retention rate after 100 cycles at 65°C (%) | Rate of increase in negative electrode thickness after 100 cycles at 65°C (%) |
|---|---|---|---|---|
| Example 1-3 | 1.1M $LiPF_6$ EC/VC/DMC/MEC (29/1/60/10) | 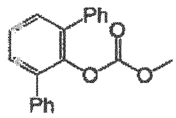 (1) | 69 | 54 |
| Example 1-4 | 1.1M $LiPF_6$ EC/VC/DMC/MEC (29/1/60/10) | 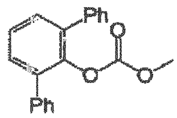 (0.3) | 67 | 59 |
| Example 1-5 | 1.1M $LiPF_6$ EC/VC/DMC/MEC (29/1/60/10) | 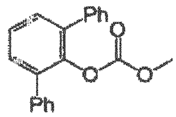 (2.5) | 68 | 58 |
| Example 1-6 | 1.1M $LiPF_6$ EC/VC/DMC/MEC (29/1/60/10) | 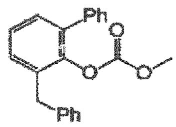 (1) | 70 | 55 |
| Example 1-7 | 1.1M $LiPF_6$ EC/VC/DMC/MEC (29/1/60/10) | 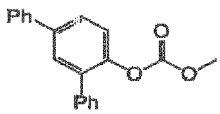 (1) | 65 | 71 |
| Example 1-8 | 1.1M $LiPF_6$ EC/VC/DMC/MEC (29/1/60/10) | 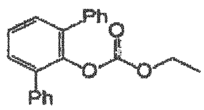 (1) | 69 | 55 |
| Example 1-9 | 1.1M $LiPF_6$ EC/VC/DMC/MEC (29/1/60/10) | 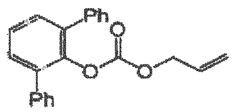 (1) | 76 | 54 |
| Example 1-10 | 1.1M $LiPF_6$ EC/VC/DMC/MEC (29/1/60/10) | 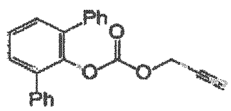 (1) | 77 | 51 |
| Example 1-11 | 1.1M $LiPF_6$ EC/VC/DMC/MEC (29/1/60/10) | 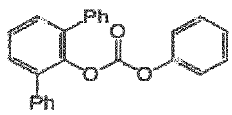 (1) | 75 | 49 |
| Example 1-12 | 1.1M $LiPF_6$ EC/FEC/VC/PC /DMC/MEC/DEC (25/3/1/1/50/15/5) | 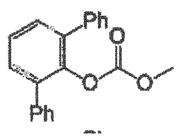 (1) | 77 | 42 |

(continued)

| | Composition of electrolyte salt Composition of nonaqueous electrolytic solution (Volume ratio of solvent) | Compound of the general formula (I) (% by mass) | Discharge capacity retention rate after 100 cycles at 65°C (%) | Rate of increase in negative electrode thickness after 100 cycles at 65°C (%) |
|---|---|---|---|---|
| Example 1-13 | 1.1M $LiPF_6$ EC/FEC/EEC/ DMC/MEC/DEC (24/5/1/50/15/5) | 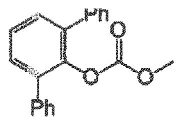 (1) | 72 | 44 |
| Comparative Example 1-1 | 1.1M $LiPF_6$ EC/DMC/MEC (30/60/10) | 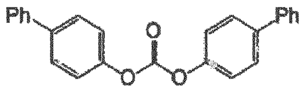 (1) | 62 | 100 |

Table 2

| | Composition of electrolyte salt Composition of nonaqueous electrolytic solution (Volume ratio of solvent) | Compound of the general formula (1) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 1-14 | 1.1M $LiPF_6$ EC/VC/DMC/MEC (29/1/60/10) | 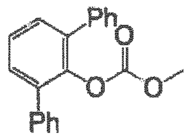 (1) | 2-Butyne-1,4-diyl dimethanesulfonate (1) + Lithium ethyl sulfate (0.2) | 78 | 39 |
| Example 1-15 | 1.1M $LiPF_6$ EC/VC/DMC/MEC (29/1/60/10) | 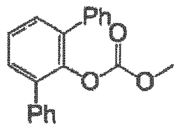 (1) | Lithium difluorophosphate (0.2) + Adiponitrile (1) | 75 | 43 |
| Example 1-16 | 1.1M $LiPF_6$ EC/VC/DMC/MEC (29/1/60/10) | 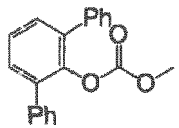 (1) | Succinic anhydride (1) + Diphenyl carbonate (0.3) | 78 | 41 |
| Example 1-17 | 1.1M $LiPF_6$ EC/VC/DMC/MEC (29/1/60/10) | 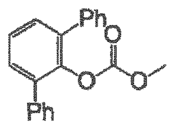 (1) | Hexamethylene diisocyanate (1) + Lithium difluoro(oxalate)borate (0.2) | 74 | 44 |
| Example 1-18 | 1.1M $LiPF_6$ EC/VC/DMC/MEC (29/1/60/10) | 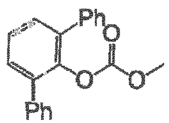 (1) | Lithium bis[oxalate-O,O']borate (0.2) + Cyclohexylbenzene (0.3) | 71 | 42 |

Table 3

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 2-1 | 1.1M $LiPF_6$ EC/VC/DMC/MEC (29/1/60/10) | 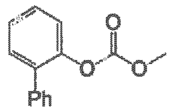 (1) | 2-Butyne-1,4-diyl dimethanesulfonate (1) | 70 | 53 |
| Example 2-2 | 1.1M $LiPF_6$ EC/VC/DMC/MEC (29/1/60/10) | 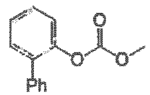 (0.3) | 2-Butyne-1,4-diyl dimethanesulfonate (3) | 68 | 58 |
| Example 2-3 | 1.1M $LiPF_6$ EC/VC/DMC/MEC (29/1/60/10) | 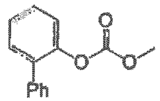 (2.5) | 2-Butyne-1,4-diyl dimethanesulfonate (0.05) | 69 | 57 |
| Example 2-4 | 1.1M $LiPF_6$ EC/VC/DMC/MEC (29/1/60/10) | 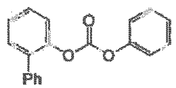 (1) | 2-Butyne-1,4-diyl dimethanesulfonate (1) | 78 | 43 |
| Example 2-5 | 1.1M $LiPF_6$ EC/VC/DMC/MEC (29/1/60/10) | 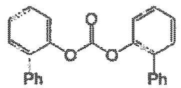 (1) | 2-Butyne-1,4-diyl dimethanesulfonate (1) | 79 | 42 |
| Example 2-6 | 1.1M $LiPF_6$ EC/VC/DMC/MEC (29/1/60/10) | 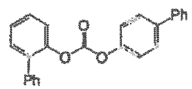 (1) | 2-Butyne-1,4-diyl dimethanesulfonate (1) | 78 | 56 |
| Example 2-7 | 1.1M $LiPF_6$ EC/FEC/EEC /DMC/MEC (24/5/1/50/15/5) | 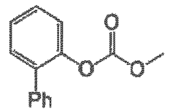 (1) | 2-Butyne-1,4-diyl dimethanesulfonate (1) | 76 | 42 |
| Example 2-8 | 1.1M $LiPF_6$ EC/FEC/VC/PC /DMC/MEC/DEC (25/3/1/1/50/15/5) | 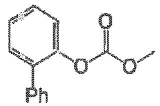 (1) | 2-Butyne-1,4-diyl dimethanesulfonate (1) | 78 | 41 |

(continued)

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Comparative Example 1-1 | 1.1M $LiPF_6$ EC/DMC/MEC (30/60/10) | 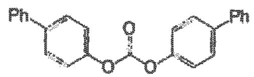 (1) | None | 62 | 100 |

Table 4

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 2-9 | 1.1M $LiPF_6$ ECA/VC/DMC/MEC (29/1/60/10) | 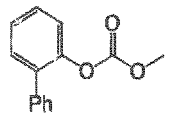 (1) | 2-Butyne-1,4-diyl dimethanesulfonate (1) + Lithium ethyl sulfate (0.2) | 80 | 39 |
| Example 2-10 | 1.1M $LiPF_6$ EC/VC/DMC/MEC (29/1/60/10) | 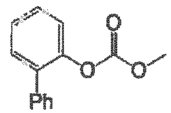 (1) | 4-Fluoro-3-trifluoromethylphenyl methanesulfonate (1) + Lithium difluorophosphate (0.2) | 78 | 39 |
| Example 2-11 | 1.1M $LiPF_6$ EC/VC/DMC/MEC (29/1/60/10) | 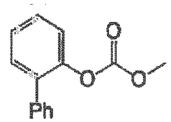 (1) | Ethyl 2-(diethoxyphosphoryl) acetate (1) + Diphenyl carbonate (0.3) | 75 | 42 |
| Example 2-12 | 1.1M $LiPF_6$ EC/VC/DMC/MEC (29/1/60/10) | 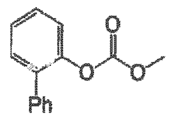 (1) | Lithium ethyl sulfate (1) + Cyclohexylbenzene (0.3) | 79 | 38 |
| Example 2-13 | 1.1M $LiPF_6$ EC/VC/DMC/MEC (29/1/60/10) | 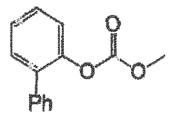 (1) | Allyl succinic anhydride (1) + Lithium bis[oxalate-O.O']borate (0.2) | 77 | 42 |
| Example 2-14 | 1.1M $LiPF_6$ EC/VC/DMC/MEC (29/1/60/10) | 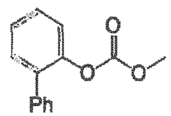 (1) | Lithium difluorobis[oxalate-O,O'] phosphate (0.2) + 1,3-Dixane (1) | 77 | 40 |

Table 5

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 2-15 | 1.1M $LiPF_6$ EC/VC/DMC/MEC (29/1/60/10) | 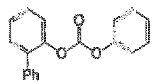 (1) | 2-Butyne-1,4-diyl dimethanesulfonate (1) + Lithium ethyl sulfate (0.2) | 81 | 36 |
| Example 2-16 | 1.1M $LiPF_6$ EC/VC/DMC/MEC (29/1/60/10) | 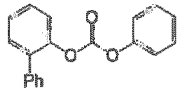 (1) | Lithium ethyl sulfate (0.2) + Adiponitrile (1) | 82 | 37 |
| Example 2-17 | 1.1M $LiPF_6$ EC/VC/DMC/MEC (29/1/60/10) | 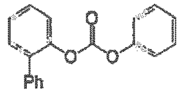 (1) | Lithium bis[oxalate-O,O']borate (0.2) + 2-Propynyl 2-(diethoxyphosphoryl)acetate (1) | 81 | 37 |
| Example 2-18 | 1.1M $LiPF_6$ EC/VC/DMC/MEC (29/1/60/10) | 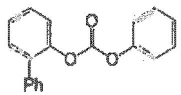 (1) | Lithium difluorobis[oxalate-O,O'] phosphate (0.2) + Hexamethylene diisocyanate (0.3) | 81 | 38 |
| Example 2-19 | 1.1M $LiPF_6$ EC/VC/DMC/MEC (29/1/60/10) | 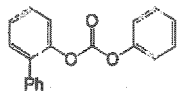 (1) | Lithium difluorobis[oxalate-O,O'] phosphate (0.2) + Diphenyl carbonate (0.3) | 82 | 36 |
| Example 2-20 | 1.1M $LiPF_6$ EC/VC/DMC/MEC (29/1/60/10) | 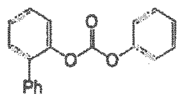 (0.5)<br>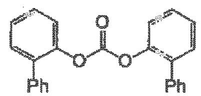 (0.5) | Lithium ethyl sulfate (0.2) + 2,2-Dioxide-1,2-oxathiolane-4-yl acetate (1) | 83 | 35 |

Examples 1-19 and Comparative Example 1-2

A negative electrode sheet was produced by using silicon (elemental substance) (negative electrode active material) in place of the negative electrode active material used in Example 1-2 and Comparative Example 1-1. 40% by mass of silicon (elemental substance), 50% by mass of artificial graphite ($d_{002}$ = 0.335 nm, negative electrode active material), and 5% by mass of acetylene black (electroconductive agent) were mixed and then added to and mixed with a solution which had been prepared by dissolving 5% by mass of polyvinylidene fluoride (binder) in 1-methyl-2-pyrrolidone in advance, thereby preparing a negative electrode mixture paste. Laminate-type batteries were produced and evaluated in the same manner as in Example 1-2 and Comparative Example 1-1, respectively, except that this negative electrode mixture paste was applied onto one surface of a copper foil (collector), dried, and treated under pressure, followed by cutting out into a predetermined size, thereby producing a negative electrode sheet. The results are shown in Table 6.

Table 6

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 1-19 | 1.1M $LiPF_6$ EC/DMC/MEC (30/60/10) | 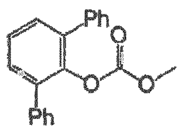 (1) | None | 58 | 44 |
| Comparative Example 1-2 | 1.1M $LiPF_6$ EC/DMC/MEC (30/60/10) | None | None | 53 | 100 |

Example 1-20 and Comparative Example 1-3

A positive electrode sheet was produced by using $LiFePO_4$ (positive electrode active material) coated with amorphous carbon in place of the positive electrode active material used in Example 1-2 and Comparative Example 1-1. 90% by mass of $LiFePO_4$ coated with amorphous carbon and 5% by mass of acetylene black (electroconductive agent) were mixed and then added to and mixed with a solution which had been prepared by dissolving 5% by mass of polyvinylidene fluoride (binder) in 1-methyl-2-pyrrolidone in advance, thereby preparing a positive electrode mixture paste. Laminate-type batteries were produced and evaluated in the same manner as in Example 1-2 and Comparative Example 1-1, respectively, except that this positive electrode mixture paste was applied onto one surface of an aluminum foil (collector), dried, and treated under pressure, followed by cutting out into a predetermined size, thereby producing a positive electrode sheet; that in evaluating the battery, the final charging voltage and the final discharging voltage were set to 3.6 V and 2.0 V, respectively; and that in Comparative Example 1-3, the compound of the general formula (I) was not added. The results are shown in Table 7.

Table 7

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 1-20 | 1.1M $LiPF_6$ EC/DMC/MEC (30/60/10) | 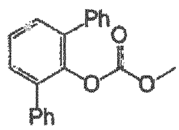 (1) | None | 70 | 56 |
| Comparative Example 1-3 | 1.1M $LiPF_6$ EC/DMC/MEC (30/60/10) | None | None | 64 | 100 |

Example 1-21 and Comparative Example 1-4

A negative electrode sheet was produced by using lithium titanate $Li_4Ti_5O_{12}$ (negative electrode active material) in place of the negative electrode active material used in Example 1-2 and Comparative Example 1-1. 80% by mass of lithium titanate $Li_4Ti_5O_{12}$ and 15% by mass of acetylene black (electroconductive agent) were mixed and then added to and mixed with a solution which had been prepared by dissolving 5% by mass of polyvinylidene fluoride (binder) in 1-methyl-2-pyrrolidone in advance, thereby preparing a negative electrode mixture paste. Laminate-type batteries were produced and evaluated in the same manner as in Example 1-2 and Comparative Example 1-1, respectively, except that this negative electrode mixture paste was applied onto one surface of a copper foil (collector), dried, and treated under pressure, followed by cutting into a predetermined size, thereby producing a negative electrode sheet; that in evaluating the battery, the final charging voltage and the final discharging voltage were set to 2.7 V and 1.2 V, respectively; and that in Comparative Example 1-4, the compound of the general formula (I) was not added. The results are shown in Table 8.

Table 8

| | Composition of electrolyte salt Composition of nonaqueous electrolytic solution (Volume ratio of solvent) | Compound of the general formula (I) (% by mass) | Compound to be used in combination with compound of the general formula (I) (% by mass) | Discharge capacity retention rate after 100 cycles at 65°C (%) | Rate of increase in negative electrode thickness after 100 cycles at 65°C (%) |
|---|---|---|---|---|---|
| Example 1-21 | 1.1M $LiPF_6$ EC/DMC/MEC (30/60/10) | 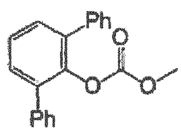 (1) | None | 76 | 72 |
| Comparative Example 1-4 | 1.1M $LiPF_6$ EC/DMC/MEC (30/60/10) | None | None | 69 | 100 |

In all of the lithium secondary batteries of Examples 1-1 to 1-18 and 2-1 to 2-20, each including the compound of the general formula (I), the increase in negative electrode thickness after high-temperature cycles is suppressed as compared with Comparative Example 1-1. In the light of the above, it has been clarified that the effect for reducing the rate of increase in electrode thickness according to the present invention is a peculiar effect to the case where the compound specified in the present invention is contained in the nonaqueous electrolytic solution having an electrolyte

salt dissolved in a nonaqueous solvent. In addition, from comparison of Example 1-19 with Comparative Example 1-2, comparison of Example 1-20 with Comparative Example 1-3, and comparison of Example 1-21 with Comparative Example 1-4, the same effect is also seen even in the case of using silicon for the negative electrode, the case of using $LiFePO_4$ for the positive electrode, and the case of using lithium titanate for the negative electrode.

Moreover, the nonaqueous electrolytic solution of the present invention also has an effect for improving discharging property of a lithium primary battery in a broad temperature range.

Industrial Applicability

By using the nonaqueous electrolytic solution of the present invention, an energy storage device which is excellent in electrochemical characteristics in a broad temperature range, especially at high temperatures, and in which an increase in electrode thickness after high-temperature cycles is suppressed, can be obtained. In particular, when the nonaqueous electrolytic solution of the present invention is used as a nonaqueous electrolytic solution for an energy storage device, such as a lithium secondary battery mounted in a device that is highly possibly used at high temperatures, e.g., a hybrid electric vehicle, a plug-in hybrid electric vehicle, a battery electric vehicle, a tablet terminal, an ultrabook, etc., an energy storage device whose electrochemical characteristics are hardly worsened in a broad temperature range, especially at high temperatures can be obtained.

## Claims

1. A nonaqueous electrolytic solution having an electrolyte salt dissolved in a nonaqueous solvent, the nonaqueous electrolytic solution comprising a biphenyl group-containing carbonate compound represented by the following general formula (I):

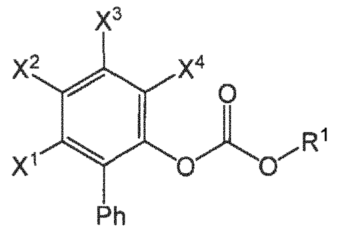

(I)

wherein $R^1$ represents an alkyl group having 1 to 12 carbon atoms, which may be substituted with a halogen atom, an alkenyl group having 2 to 6 carbon atoms, an alkynyl group having 3 to 6 carbon atoms, or an aryl group having 6 to 20 carbon atoms, which may be substituted with a halogen atom; and each of $X^1$ to $X^4$ independently represents a hydrogen atom, a phenyl group, or a benzyl groups,
wherein,
the nonaqueous electrolytic solution further comprises a fluorine atom-containing cyclic carbonate or an unsaturated bond-containing cyclic carbonate.

2. The nonaqueous electrolytic solution according to claim 1, wherein in the general formula (I), at least one of $X^1$ to $X^4$ is a phenyl group or a benzyl group.

3. The nonaqueous electrolytic solution according to claim 2, wherein the compound represented by the general formula (I) is one or more selected from 2,6-diphenylphenyl methyl carbonate, 2,6-diphenylphenyl ethyl carbonate, 2,6-diphenylphenyl allyl carbonate, 2,6-diphenylphenyl 2-propynyl carbonate, 2,6-diphenylphenyl phenyl carbonate, 2,6-diphenylphenyl 2-phenylphenyl carbonate, 2,6-diphenylphenyl 3-phenylphenyl carbonate, 2,6-diphenylphenyl 4-phenylphenyl carbonate, 2,4-diphenylphenyl methyl carbonate, 2-benzyl-6-phenylphenyl methyl carbonate, 2-benzyl-6-phenylphenyl ethyl carbonate, 2-benzyl-6-phenylphenyl allyl carbonate, 2-benzyl-6-phenylphenyl 2-propynyl carbonate, 2-benzyl-6-phenylphenyl phenyl carbonate, 2-benzyl-6-phenylphenyl 2-phenylphenyl carbonate, 2-benzyl-6-phenylphenyl 3-phenylphenyl carbonate, and 2-benzyl-6-phenylphenyl 4-phenylphenyl carbonate.

4. The nonaqueous electrolytic solution according to claim 1, wherein in the general formula (I), all of $X^1$ to $X^4$ are a hydrogen atom.

5. The nonaqueous electrolytic solution according to claim 4, wherein the compound represented by the general formula

(I) is one or more selected from methyl 2-phenylphenyl carbonate, ethyl 2-phenylphenyl carbonate, allyl 2-phenylphenyl carbonate, 2-phenylphenyl 2-propynyl carbonate, phenyl 2-phenylphenyl carbonate, bis(2-phenylphenyl) carbonate, 2-phenylphenyl 3-phenylphenyl carbonate, and 2-phenylphenyl 4-phenylphenyl carbonate.

**6.** The nonaqueous electrolytic solution according to any one of claims 1 to 5, wherein the nonaqueous solvent comprises a cyclic carbonate and a linear carbonate, and the linear carbonate comprises both a symmetric linear carbonate and an asymmetric linear carbonate.

**7.** The nonaqueous electrolytic solution according to claim 6, wherein the asymmetric linear carbonate is at least one selected from methyl ethyl carbonate, methyl propyl carbonate, methyl isopropyl carbonate, methyl butyl carbonate, and ethyl propyl carbonate.

**8.** The nonaqueous electrolytic solution according to claim 6 or 7, wherein the cyclic carbonate comprises at least two or more selected from ethylene carbonate, propylene carbonate, 1,2-butylene carbonate, and 2,3-butylene carbonate.

**9.** The nonaqueous electrolytic solution according to any one of claims 1 to 8, further comprising at least one selected from an $SO_2$ group-containing compound, a fluorinated benzene compound, a phosphoric acid ester compound, a carbon-carbon triple bond-containing compound, a carboxylic acid anhydride, an isocyanate compound, a lithium-containing ionic compound, a nitrile compound, a benzene compound, a cyclic acetal compound, and a phosphazene compound.

**10.** The nonaqueous electrolytic solution according to any one of claims 1 to 9, wherein the electrolyte salt is one or more lithium salts selected from $LiPF_6$, $LiBF_4$, $LiN(SO_2CF_3)_2$, and $LiN(SO_2F)_2$.

**11.** The nonaqueous electrolytic solution according to claim 10, wherein the concentration of the lithium salt is 1.1 M or more relative to the nonaqueous solvent.

**12.** The nonaqueous electrolytic solution according to any one of claims 1 to 11, further comprising a lithium-containing ionic compound which is one or more selected from lithium difluorophosphate, lithium fluorophosphate, lithium fluorosulfonate, lithium difluorobis[oxalate-O,O’]phosphate, lithium tetrafluoro[oxalate-O,O’]phosphate, lithium bis[oxalate-O,O’]borate, lithium difluoro[oxalate-O,O’]borate, lithium methyl sulfate, lithium ethyl sulfate, and lithium propyl sulfate.

**13.** The nonaqueous electrolytic solution according to any one of claims 1 to 12, further comprising a nitrile compound which is one or more selected from acetonitrile, propionitrile, succinonitrile, glutaronitrile, adiponitrile, pimelonitrile, suberonitrile, and sebaconitrile.

**14.** An energy storage device comprising a positive electrode, a negative electrode, and a nonaqueous electrolytic solution having an electrolyte salt dissolved in a nonaqueous solvent, wherein the nonaqueous electrolytic solution is the nonaqueous electrolytic solution according to any one of claims 1 to 13.

**15.** The energy storage device according to claim 14, wherein an active material of the positive electrode is a complex metal oxide containing lithium and one or more selected from cobalt, manganese, and nickel, or a lithium-containing olivine-type phosphate containing one or more selected from iron, cobalt, nickel, and manganese.

## Patentansprüche

**1.** Nicht-wässrige Elektrolytlösung mit einem Elektrolytsalz, gelöst in einem nicht-wässrigen Lösungsmittel, wobei die nicht-wässrige Elektrolytlösung eine eine Biphenylgruppe enthaltende Carbonatverbindung der folgenden allgemeinen Formel (I) umfasst:

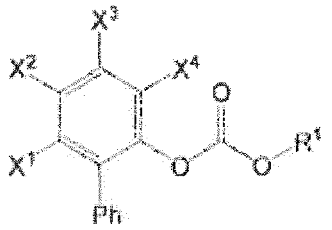

(I)

worin $R^1$ eine Alkylgruppe mit 1 bis 12 Kohlenstoffatomen darstellt, die mit einem Halogenatom, einer Alkenylgruppe mit 2 bis 6 Kohlenstoffatomen, einer Alkinylgruppe mit 3 bis 6 Kohlenstoffatomen oder einer Arylgruppe mit 6 bis 20 Kohlenstoffatomen, die mit einem Halogenatom substituiert sein kann, substituiert sein kann; und jedes von $X^1$ bis $X^4$ stellt unabhängig voneinander ein Wasserstoffatom, eine Phenylgruppe oder eine Benzylgruppe dar, worin

die nicht-wässrige Elektrolytlösung desweiteren ein ein Fluoratom-enthaltendes cyclisches Carbonat oder ein eine ungesättigte Bindung enthaltendes cyclisches Carbonat umfasst.

**2.** Nicht-wässrige Elektrolytlösung nach Anspruch 1, worin in der allgemeinen Formel (I) mindestens eines von $X^1$ bis $X^4$ eine Phenylgruppe oder eine Benzylgruppe ist.

**3.** Nicht-wässrige Elektrolytlösung nach Anspruch 2, worin die durch die allgemeine Formel (I) dargestellte Verbindung eine oder mehrere ist, ausgewählt aus 2,6-Diphenylphenylmethylcarbonat, 2,6-Diphenylphenylethylcarbonat, 2,6-Diphenylphenylallylcarbonat, 2,6-Diphenylphenyl-2-propinylcarbonat, 2,6-Diphenylphenylphenylcarbonat, 2,6-Diphenylphenyl-2-phenylphenylcarbonat, 2,6-Diphenylphenyl-3-phenylphenylcarbonat, 2,6-Diphenylphenyl-4-phenylphenylcarbonat, 2-Benzyl-6-phenylphenylmethylcarbonat, 2-Benzyl-6-phenylphenylethylcarbonat, 2-Benzyl-6-phenylphenylallylcarbonat, 2-Benzyl-6-phenylphenyl-2-propynylcarbonat, 2-Benzyl-6-phenylphenylphenylcarbonat, 2-Benzyl-6-phenylphenyl-2-phenylphenylcarbonat, 2-Benzyl-6-phenylphenyl-3-phenylphenylcarbonat und 2-Benzyl-6-phenylphenyl-4-phenylphenylcarbonat.

**4.** Nicht-wässrige Elektrolytlösung nach Anspruch 1, worin in der allgemeinen Formel (I) alle $X^1$ bis $X^4$ ein Wasserstoffatom sind.

**5.** Nicht-wässrige Elektrolytlösung nach Anspruch 4, worin die durch die allgemeine Formel (I) dargestellte Verbindung eine oder mehrere ist, ausgewählt aus Methyl-2-phenylphenylcarbonat, Ethyl-2-phenylphenylcarbonat, Allyl-2-phenylphenylcarbonat, 2-Phenylphenyl-2-propinylcarbonat, Phenyl-2-phenylphenylcarbonat, Bis(2-phenylphenyl)carbonat, 2-Phenylphenyl-3-phenylphenylcarbonat und 2-Phenylphenyl-4-phenylphenylcarbonat.

**6.** Nicht-wässrige Elektrolytlösung nach einem der Ansprüche 1 bis 5, worin das nicht-wässrige Lösungsmittel ein cyclisches Carbonat und ein lineares Carbonat umfasst und das lineare Carbonat sowohl ein symmetrisches lineares Carbonat als auch ein asymmetrisches lineares Carbonat umfasst.

**7.** Nicht-wässrige Elektrolytlösung nach Anspruch 6, worin das asymmetrische lineare Carbonat mindestens eines ist, ausgewählt aus Methylethylcarbonat, Methylpropylcarbonat, Methylisopropylcarbonat, Methylbutylcarbonat und Ethylpropylcarbonat.

**8.** Nicht-wässrige Elektrolytlösung nach Anspruch 6 oder 7, worin das cyclische Carbonat mindestens zwei oder mehr umfasst, ausgewählt aus Ethylencarbonat, Propylencarbonat, 1,2-Butylencarbonat und 2,3-Butylencarbonat.

**9.** Nicht-wässrige Elektrolytlösung nach einem der Ansprüche 1 bis 8, desweiteren umfassend mindestens eine Verbindung, ausgewählt aus einer $SO_2$-Gruppen enthaltenden Verbindung, einer fluorierten Benzolverbindung, einer Phosphorsäureesterverbindung, einer eine Kohlenstoff-Kohlenstoff-Dreifachbindung enthaltenden Verbindung, einem Carbonsäureanhydrid, einer Isocyanatverbindung, einer lithiumhaltigen ionischen Verbindung, einer Nitrilverbindung, einer Benzolverbindung, einer cyclischen Acetalverbindung und einer Phosphazenverbindung.

**10.** Nicht-wässrige Elektrolytlösung nach einem der Ansprüche 1 bis 9, worin das Elektrolytsalz ein oder mehrere Lithiumsalze ist, ausgewählt aus $LiPF_6$, $LiBF_4$, $LiN(SO_2CF_3)_2$ und $LiN(SO_2F)_2$.

**11.** Nicht-wässrige Elektrolytlösung nach Anspruch 10, worin die Konzentration des Lithiumsalzes 1,1 M oder mehr,

bezogen auf das nicht-wässrige Lösungsmittel, beträgt.

**12.** Nicht-wässrige Elektrolytlösung nach einem der Ansprüche 1 bis 11, umfassend desweiteren eine lithiumhaltige ionische Verbindung, die eine oder mehrere ist, ausgewählt aus Lithiumdifluorphosphat, Lithiumfluorphosphat, Lithiumfluorsulfonat, Lithiumdifluorbis[oxalat-O,O']phosphat, Lithiumtetrafluor[oxalat-O,O']phosphat, Lithiumbis[oxalat-O,O']borat, Lithiumdifluor[oxalat-O,O']borat, Lithiummethylsulfat, Lithiumethylsulfat und Lithiumpropylsulfat.

**13.** Nicht-wässrige Elektrolytlösung nach einem der Ansprüche 1 bis 12, umfassend desweiteren eine Nitrilverbindung, die eine oder mehrere ist, ausgewählt aus Acetonitril, Propionitril, Succinonitril, Glutaronitril, Adiponitril, Pimelonitril, Suberonitril und Sebaconitril.

**14.** Energiespeichervorrichtung, umfassend eine positive Elektrode, eine negative Elektrode und eine nicht-wässrige Elektrolytlösung mit einem Elektrolytsalz, gelöst in einem nicht-wässrigen Lösungsmittel, worin die nicht-wässrige Elektrolytlösung die nicht-wässrige Elektrolytlösung nach einem der Ansprüche 1 bis 13 ist.

**15.** Energiespeichervorrichtung nach Anspruch 14, worin ein aktives Material der positiven Elektrode ein komplexes Metalloxid ist, enthaltend Lithium und eines oder mehrere ausgewählt aus Kobalt, Mangan und Nickel, oder ein Lithium-enthaltendes Olivin-Phosphat, enthaltend eines oder mehrere ausgewählt aus Eisen, Kobalt, Nickel und Mangan.

## Revendications

**1.** Solution électrolytique non aqueuse ayant un sel d'électrolyte dissous dans un solvant non aqueux, la solution électrolytique non aqueuse comprenant un composé de carbonate contenant un groupe biphényle représenté par la formule générale (I) suivante :

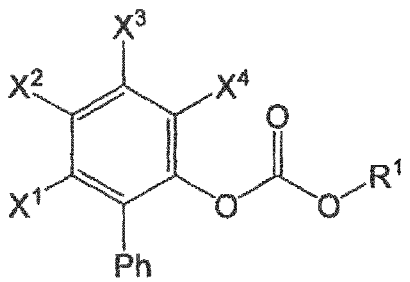

(I)

dans laquelle $R^1$ représente un groupe alkyle ayant 1 à 12 atome(s) de carbone, qui peut être substitué par un atome d'halogène, un groupe alcényle ayant 2 à 6 atomes de carbone, un groupe alcynyle ayant 3 à 6 atomes de carbone ou un groupe aryle ayant 6 à 20 atomes de carbone, qui peut être substitué par un atome d'halogène ; et chacun de $X^1$ à $X^4$ représente indépendamment un atome d'hydrogène, un groupe phényle ou un groupe benzyle, dans laquelle,
la solution électrolytique non aqueuse comprend en outre un carbonate cyclique contenant un atome de fluor ou un carbonate cyclique contenant une liaison insaturée.

**2.** Solution électrolytique non aqueuse selon la revendication 1, dans laquelle dans la formule générale (I), au moins l'un de $X^1$ à $X^4$ représente un groupe phényle ou un groupe benzyle.

**3.** Solution électrolytique non aqueuse selon la revendication 2, dans laquelle le composé représenté par la formule générale (1) est un ou plusieurs composé(s) choisi(s) parmi le méthylcarbonate de 2,6-diphénylphényle, l'éthylcarbonate de 2,6-diphénylphényle, l'allylcarbonate de 2,6-diphénylphényle, le 2-propynylcarbonate de 2,6-diphénylphényle, le phénylcarbonate de 2,6-diphénylphényle, le 2-phénylphénylcarbonate de 2,6-diphénylphényle, le 3-phénylphénylcarbonate de 2,6-diphénylphényle, le 4-phénylphénylcarbonate de 2,6-diphénylphényle, le méthylcarbonate de 2,4-diphénylphényle, le méthylcarbonate de 2-benzyl-6-phénylphényle, l'éthylcarbonate de 2-benzyl-6-phénylphényle, l'allylcarbonate de 2-benzyl-6-phénylphényle, le 2-propynylcarbonate 2-benzyl-6-phénylphényle, le phénylcarbonate de 2-benzyl-6-phénylphényle, le 2-phénylphénylcarbonate de 2-benzyl-6-phénylphényle, le 3-phénylphénylcarbonate de 2-benzyl-6-phénylphényle et le 4-phénylphénylcarbonate de 2-benzyl-6-phénylphényle.

4. Solution électrolytique non aqueuse selon la revendication 1, dans laquelle dans la formule générale (I), tous les $X^1$ à $X^4$ représentent un atome d'hydrogène.

5. Solution électrolytique non aqueuse selon la revendication 4, dans laquelle le composé représenté par la formule générale (I) est un ou plusieurs composé(s) choisi(s) parmi le 2-phénylphénylcarbonate de méthyle, le 2-phénylphénylcarbonate d'éthyle, le 2-phénylphénylcarbonate d'allyle, le 2-propynylcarbonate de 2-phénylphényle, le 2-phénylphénylcarbonate de phényle, le carbonate de bis(2-phénylphényle), le 3-phénylphénylcarbonate de 2-phénylphényle et le 4-phénylphénylcarbonate de 2-phénylphényle.

6. Solution électrolytique non aqueuse selon l'une quelconque des revendications 1 à 5, dans laquelle le solvant non aqueux comprend un carbonate cyclique et un carbonate linéaire, et le carbonate linéaire comprend à la fois un carbonate linéaire symétrique et un carbonate linéaire asymétrique.

7. Solution électrolytique non aqueuse selon la revendication 6, dans laquelle le carbonate linéaire asymétrique est au moins un composé choisi parmi le carbonate de méthyléthyle, le carbonate de méthylpropyle, le carbonate de méthylisopropyle, le carbonate de méthylbutyle et le carbonate d'éthylpropyle.

8. Solution électrolytique non aqueuse selon la revendication 6 ou 7, dans laquelle le carbonate cyclique comprend au moins deux composés ou plus choisis parmi le carbonate d'éthylène, le carbonate de propylène, le carbonate de 1,2-butylène et le carbonate de 2,3-butylène.

9. Solution électrolytique non aqueuse selon l'une quelconque des revendications 1 à 8, comprenant en outre au moins un composé choisi parmi un composé contenant un groupe $SO_2$, un composé de benzène fluoré, un composé d'ester d'acide phosphorique, un composé contenant une triple liaison carbone-carbone, un anhydride d'acide carboxylique, un composé d'isocyanate, un composé ionique contenant du lithium, un composé de nitrile, un composé de benzène, un composé d'acétal cyclique et un composé de phosphazène.

10. Solution électrolytique non aqueuse selon l'une quelconque des revendications 1 à 9, dans laquelle le sel d'électrolyte est un ou plusieurs sel(s) de lithium choisis parmi $LiPF_6$, $LiBF_4$, $LiN(SO_2CF_3)_2$ et $LiN(SO_2F)_2$.

11. Solution électrolytique non aqueuse selon la revendication 10, dans laquelle la concentration du sel de lithium est supérieure ou égale à 1,1 M par rapport au solvant non aqueux.

12. Solution électrolytique non aqueuse selon l'une quelconque des revendications 1 à 11, comprenant en outre un composé ionique contenant du lithium qui est un ou plusieurs composé(s) choisi(s) parmi le difluorophosphate de lithium, le fluorophosphate de lithium, le fluorosulfonate de lithium, le difluorobis[oxalate-O,O']phosphate de lithium, le tétrafluoro[oxalate-O,O']phosphate de lithium, le bis[oxalate-O,O']borate de lithium, le difluoro[oxalate-O,O']borate de lithium, le méthylsulfate de lithium, l'éthylsulfate de lithium et le propylsulfate de lithium.

13. Solution électrolytique non aqueuse selon l'une quelconque des revendications 1 à 12, comprenant en outre un composé de nitrile qui est un ou plusieurs composé(s) choisi(s) parmi l'acétonitrile, le propionitrile, le succinonitrile, le glutaronitrile, l'adiponitrile, le pimélonitrile, le subéronitrile et le sébaconitrile.

14. Dispositif de stockage d'énergie comprenant une électrode positive, une électrode négative et une solution électrolytique non aqueuse ayant un sel d'électrolyte dissous dans un solvant non aqueux, dans lequel la solution électrolytique non aqueuse est la solution électrolytique non aqueuse selon l'une quelconque des revendications 1 à 13.

15. Dispositif de stockage d'énergie selon la revendication 14, dans lequel un matériau actif de l'électrode positive est un oxyde métallique complexe contenant du lithium et un ou plusieurs élément(s) choisi(s) parmi le cobalt, le manganèse et le nickel, ou un phosphate de type olivine contenant du lithium contenant un ou plusieurs élément(s) choisi(s) parmi le fer, le cobalt, le nickel et le manganèse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004111169 A